# EUROPEAN PATENT SPECIFICATION

(11) **EP 2 640 368 B1**
(45) Date of publication and mention of the grant of the patent: **30.12.2020**
(21) Application number: 11842396.1
(22) Date of filing: 15.11.2011
(51) Int. Cl.: A61K 47/28, A61K 31/07, A61K 47/10, A61K 47/24, A61K 47/32, A61K 9/70, A61P 35/00

(54) **CONTROLLED RELEASE MUCOADHESIVE SYSTEMS**
MUKOADHÄSIVE SYSTEME MIT KONTROLLIERTER FREISETZUNG
SYSTÈMES MUCOADHÉSIFS À LIBÉRATION CONTRÔLÉE

(30) Priority: 15.11.2010 US 413982 P
(43) Date of publication of application: 25.09.2013
(73) Proprietor: The Ohio State University Research Foundation, Columbus, OH 43201 (US); The Regents of The University of Michigan, Ann Arbor, Michigan 48109 (US)
(72) Inventor: MALLERY, Susan, R., Columbus OH 43214 (US); LARSEN, Peter, E., Powell OH 43065 (US); STONER, Gary, D., Worthington OH 43085 (US); SCHWENDEMAN, Steven, P., Superior Twp. MI 48198 (US); DESAI, Kashappa-Goud, MI 48104 (US)
(74) Representative: Turner, Craig Robert
(86) International application number: PCT/US2011/060838
(87) International publication number: WO 2012/068147

(56) References cited:
- WO-A2-2005/009342
- US-A1- 2003 206 958
- US-A1- 2005 106 216
- US-A1- 2007 015 841
- US-B2- 7 790 905
- US-B2- 7 790 905

## Description

### TECHNICAL FIELD AND INDUSTRIAL APPLICABILITY OF THE INVENTION

This invention is directed to formulations for chemoprevention of oral cancer and precancerous lesions, and for methods for preparing the formulations.

Specifically, the invention relates to adhesive gels containing a hydrophobic formulation (such as fenretinide), formulated for local delivery for the chemoprevention of oral cancer and precancerous lesions. The invention relates also to methods for stabilizing and enhancing the efficacy of chemopreventive components of the formulations.

### BACKGROUND OF THE INVENTION

Head and neck squamous cell carcinoma (HNSCC), which is a world-wide health problem, will affect approximately 36,000 Americans with over 7,000 deaths this year. Despite extensive research and introduction of therapeutic advances such as radiation-intensification, prognosis for persons with HNSCC remains among the lowest of all solid tumors.

Intervention with effective chemopreventive agents-to prevent progression or induce regression- at the pre-cancerous stage would greatly improve clinical outcomes. Analogous to other surface origin malignancies, initiated head and neck epithelium undergoes progressive growth disturbances (grades of epithelial dysplasia) prior to conversion to overt carcinoma. Furthermore, many of these dysplastic lesions arise in visible mucosa, making topical application and direct clinical monitoring of lesion progression feasible. Despite obtaining complete surgical excision, many of these dysplastic lesions recur; necessitating sequential surgeries and increasing patient anxieties regarding cancer development.

The buccal mucosa is an attractive site for the localized delivery of therapeutic agents to treat or prevent oral cancer by using a mucoadhesive patch. However, the benefits of this administration route may be limited due to the barrier properties of the buccal mucosa. Since the buccal mucosa is a tissue that is exposed to many foreign agents, the buccal mucosa significant barrier properties may hinder the transport of therapeutically active compounds.

For example, small lipophilic drug molecules with a log P of 1.6 - 3.3 are generally believed to permeate well because of greater partitioning into the tissue. However, for highly lipophilic drugs with a log P greater than 3.5, a decrease in permeability is observed due to their limited water solubility. In fact, most agents that are better known for enhancing drug permeability through the skin also improve the transport of compounds across the buccal mucosa.

To increase the permeation of drugs, chemical approaches such as the utilization of chemical permeation enhancers (e.g., surfactants, bile salts, and fatty acids) might be only applicable to patch preparations. There is still a need, however, for formulations that can readily cross the buccal mucosa barriers.

### SUMMARY

According to one aspect of the present invention, there is provided a formulation, comprising: at least one mucoadhesive material; at least an effective amount of fenretinide or a pharmaceutically acceptable salt thereof; and, at least one transmucosal permeation enhancer agent comprising: propylene glycol (PG) and menthol; and at least one solubilizer agent.

Also disclosed herein is a formulation, comprising: at least one mucoadhesive material; at least one retinide composition or a pharmaceutically acceptable salt thereof; and, at least one transmucosal permeation enhancer agent selected for enhancing permeation of the retinide composition across a mucosa; and; optionally, at least one solubilizer agent for enhancing release of the retinide composition from the mucoadhesive material.

Also disclosed herein is a formulation, comprising: at least one mucoadhesive material; at least one retinide therapeutic agent; and, at least one transmucosal permeation enhancer agent selected from one or more of: propylene glycol (PG) and a terpene or terpenoid composition; and; optionally, at least one solubilizer agent

Also disclosed herein is a formulation, comprising: at least an effective amount of a pharmaceutically active fenretinide composition, and at least one transmucosal permeation enhancer agent selected from propylene glycol (PG) and menthol.

Also disclosed herein is a formulation, comprising: at least an effective amount of a pharmaceutically active fenretinide composition, and transmucosal permeation enhancer agents comprising propylene glycol (PG) and menthol.

In certain embodiments, the formulation comprises from about 1 wt% to about 2.5 wt% PG, and from 1 wt % to about 5 wt% menthol.

In certain embodiments, the formulation comprises from about 1 wt % to about 2.5 wt% PG, and about 5 wt% menthol.

In certain embodiments, the formulation comprises about 1 wt% PG and about 5 wt% menthol.

In certain embodiments, the formulation comprises about 2.5 wt% PG and about 5 wt% menthol.

In certain embodiments, the pharmaceutically active fenretinide composition and the at least one permeation enhancer agent are adapted to be in contact with at least on common mucosal membrane.

Optionally, the mucosal membrane is the buccal mucosa.

According to another aspect of the present invention, there is provided a transmucosal drug delivery system comprising at least drug release layer comprised of the formulation of the present invention, at least one adhesive layer, and at least one backing layer.

According to another aspect of the present invention, there is provided the formulation of the present invention for use in a method, comprising: i) providing a transmucosal system comprising the formulation of the present invention; ii) applying the transmucosal system to a mucosal membrane of a subject, optionally the buccal mucosa; and, iii) keeping the transmucosal system in contact with the mucosal membrane for a therapeutically effective period of time; and, iv) optionally removing the transmucosal system when a desired therapeutic effect has been achieved.

In certain embodiments, the transmucosal system includes an adhesive material.

Optionally, the formulation and the adhesive material are present in separate compartments.

According to another aspect of the present invention, there is provided the formulation of the present invention for use in a method of treatment and prophylaxis of a disease or disorder, said use comprising: administering said formulation to a subject in need of such treatment.

Also disclosed herein is a formulation for application to the oral mucosa, comprising: at least an effective amount of a pharmaceutically active fenretinide composition, and at least one transmucosal permeation enhancer agent selected from propylene glycol (PG) and menthol.

Also disclosed herein is a method for treating or preventing head and neck squamous cell carcinoma (HNSCC), comprising administering to a subject an effective amount of a formulation generally described herein.

Also disclosed herein is a drug dosage form for oral transmucosal administration, comprising the formulation generally described herein. The drug dosage form may further include an adhesive material, the adhesive material providing for adherence to the oral mucosal membrane of a subject. The oral mucosal membrane may be a buccal membrane.

According to another aspect of the present invention, there is provided a drug dosage form for oral transmucosal administration, comprising a formulation comprised of: at least an effective amount of fenretinide or a pharmaceutically acceptable salt thereof, and at least one transmucosal permeation enhancer agent selected from propylene glycol (PG) and menthol; and, an adhesive material, the adhesive material providing for adherence of the drug dosage form to the oral mucosal membrane.

In certain embodiments, the formulation contains a predetermined amount of pharmaceutically active fenretinide composition in an amount selected from the group consisting of 10 µg, 15 µg, 25 µg, 50 µg, 100 µg, 500 µg, 1 mg, 2 mg, 3 mg, 4 mg, 5 mg, 6 mg, 7 mg, 8 mg, 9 mg and 10 mg and an adhesive material, the adhesive material providing for adherence to the oral mucosal membrane of a subject.

In certain embodiments, the amount of the pharmaceutically active fenretinide absorbed via the oral mucosa is selected from the group consisting of at least 35%, at least 40%, at least 45%, at least 50%, at least 55%, at least 60%, at least 65%, at least 70%, at least 75%, at least 80%, at least 85%, at least 90%, at least 95%, at least 98% and at least 99% of the drug in the dosage form.

In certain embodiments, a single or repeated oral transmucosal administration to a subject results in a bioavailability of greater than 70%, greater than 75%., greater than 80%, greater than 85%, greater than 90%, or greater than 94%.

In certain embodiments, a single or repeated oral transmucosal administration to a subject results in a bioavailability with a coefficient of variation of less than 30%, or less than 40%.

In certain embodiments, a single oral transmucosal administration of the drug dosage form to a subject results in a Tₘₐₓ of from about 6 hours to about 12 hours.

In certain embodiments, a single oral transmucosal administration of the drug dosage form to a subject results in a Tₘₐₓ of from about 6 hours to about 8 hours.

Also disclosed herein is a method of treating a subject exhibiting a symptomatic medical condition, comprising, administering the formulation generally described herein in a pharmaceutically active amount of a drug effective to reduce or eliminate the symptoms in the subject. The symptomatic medical condition may be an oral cancer or pre-cancerous condition.

Also disclosed herein is a method of a treating an oral cancer or a precancerous condition in a subject comprising, administering the formulation generally described herein.

According to another aspect of the present invention, there is provided a method for making the formulation of the present invention, comprising: i) mixing a quantity of at least one solubilizer, at least one mucoadhesive polymer, and at least one permeation enhancer in a solvent to form a solvent mixture; ii) adding a quantity of fenretinide to the solvent mixture of step i); and, optionally adjusting a volume thereof to 10 mL with the solvent mixture of step i); iii) forming a layer of the fenretinide mixture of step ii); and, iv) drying the layer of step iii).

In certain embodiments, the solubilizer comprises one or more of: polysorbate 80 (brand names include Alkest®, Canarcel® and Tween® 80 (a registered trademark of ICI Americas, Inc)) which is a nonionic surfactant and emulsifier derived from polyethoxylated sorbitan and oleic acid, and sodium deoxycholate.

The permeation enhancer used in the method for making the formulation of the present invention comprises propylene glycol and menthol.

In certain embodiments, the formulation of the present invention is used in a method for chemoprevention of an oral cancer or precancerous condition, said use comprising topically administering to a subject in need of such chemoprevention the formulation generally described herein.

Optionally, the formulation is administered to an interior of an oral cavity of the subject.

Also disclosed herein is a mucoadhesive system useful for intraoral administration and slow release of a highly hydrophobic formulation, comprising a co-solvent system that enhances oral mucosal permeation of a hydrophobic formulation.

Also disclosed herein are formulations comprising a highly hydrophobic chemopreventive agent. The formulations disclosed herein may comprise a retinide composition, such as fenretinide.

Described herein is a method for enhancing oral mucosal permeation of a hydrophobic formulation, comprising co-incorporation of a co-solvent in a hydrogel-based controlled release system.

The method disclosed above may include forming a drug layer comprising fenretinide, mucoadhesive material Eudragit® RL PO films containing mixed nonionic surfactants and deoxycholate solubilizers. The drug layer may comprise one or more of: nonionic surfactants, bile salts, phospholipids, and polymeric solubilizers.

Described herein is a formulation for chemoprevention of an oral cancer or precancerous condition, comprising a hydrogel-based controlled release mucoadhesive system.

The formulations disclosed herein may comprise at least one therapeutic agent in an amount effective for chemoprevention.

The adhesive carrier may be a mucoadhesive gel adapted for transmucosal delivery of fenretinide.

Described herein is a method for chemoprevention of an oral cancer or precancerous condition, comprising topically administering to a subject in need of such chemoprevention a solublized fenretinide preparation admixed with a permeation enhancer agent.

The fenretinide preparation admixed with the permeation enhancer agent may be administered to an interior of an oral cavity of the subject.

The permeation enhancers may comprise a mixture of propylene glycol, L-menthol and oleic acid.

Described herein is a method for increasing the concentration of a hydrophobic therapeutic agent in a bodily tissue or fluid of a subject at risk of an oral cancer or precancerous condition, comprising applying a preparation containing the therapeutic agent to an interior of an oral cavity of the subject.

The bodily tissue or fluid may be selected from the group consisting of a mucosal tissue, an oral mucosa tissue, oral tissue, peripheral blood, serum, and saliva.

Described herein is a method for preparing a formulation for chemoprevention of an oral cancer or precancerous condition.

Described herein is a method for improving the efficacy of a formulation for chemoprevention of an oral cancer or precancerous condition, comprising: providing a fenretinide preparation; admixing the fenretinide preparation with a permeation enhancer formulation to form a mixture; prior to applying the mixture to an oral cavity of a subject in need of chemoprevention.

According to another aspect of the present invention there is provided a method for making a buccal drug delivery system, comprising: preparing a drug-release layer comprised of the formulation of the present invention; preparing an adhesive layer; and, assembling the drug layer and the adhesive layers onto a backing layer.

Described herein is a formulation comprising a solubilizer agent for enhancing release of fenretinide from the patch; and, a permeation enhancer agent for improving permeation of fenretinide across a mucosa.

Described herein is a method for increasing release of a retinide composition from a drug-release layer, comprising: admixing a retinide composition with a solubilizer, and forming the admixture into a drug-release layer.

According to another aspect of the present invention, there is provided a method for increasing permeation of fenretinide into a mucosa of a subject in need thereof, the drug dosage form being prepared by: i) admixing fenretinide with a permeation enhancer agent comprised of one or more of propylene glycol and menthol, and ii) forming the admixture into a drug-release layer.

Described herein is a method for increasing release of a retinide composition from a drug-release layer and for increasing permeation of the retinide composition into a mucosa of a subject in need thereof, comprising: admixing a retinide composition with a solubilizer and a permeation enhancer agent, and forming the admixture into a drug-release layer.

Described herein is a method of treatment and prophylaxis of a disease comprising administering to a subject in need of such treatment the formulation.

Described herein is a method for increasing the concentration of a retinide composition in a bodily tissue or fluid of a subject at risk of an oral cancer or precancerous condition, comprising applying the formulation to an interior of an oral cavity of the subject. The bodily tissue or fluid may be selected from the a mucosal tissue, an oral mucosa tissue and oral tissue

### BRIEF DESCRIPTION OF THE DRAWINGS

The patent or application file contains one or more drawings executed in color and/or one or more photographs. Copies of this patent or patent application publication with color drawing(s) and/or photograph(s) will be provided by the Patent Office upon request and payment of the necessary fee.

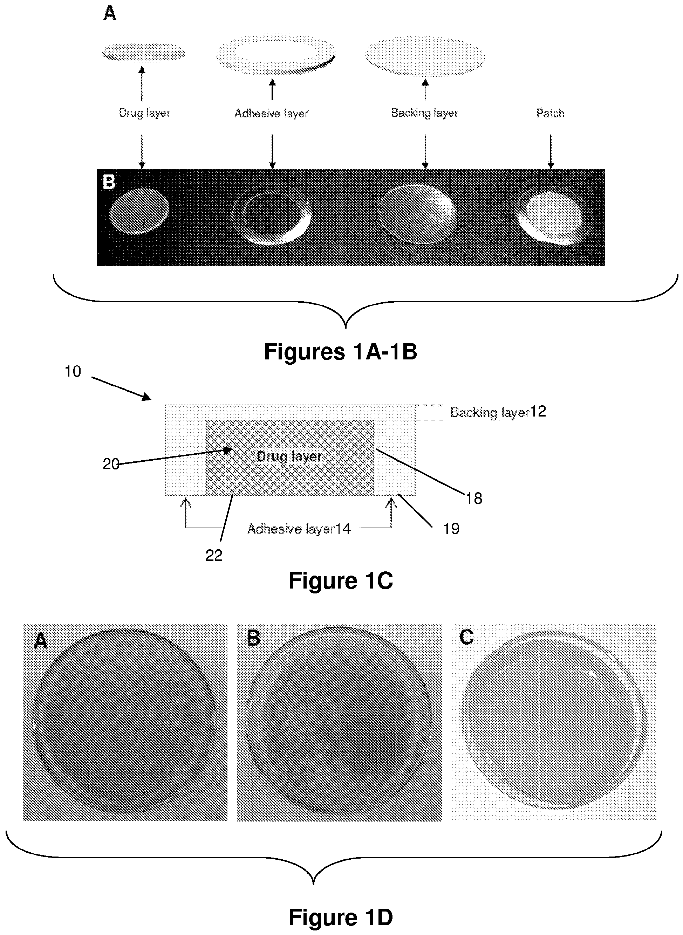

**Figures 1A****-C:** Schematic diagrams **(****Figure 1A****),** photographic image **(****Figure 1B**), and schematic cross-sectional diagram **(****Figure 1C****)** of a mucoadhesive patch comprising drug (fenretinide-mucoadhesive material (Eudragit® RL PO) with or w/o solubilizer), adhesive (hydroxypropyl methylcellulose (HPMC) 4KM: polycarbophil (3:1), and backing (Tegaderm™ dressing) layers.
**Figure 1D****:** Photographs of the fenretinide/Eudragit® (drug release) layer loaded with 5 wt% menthol (photograph A), 10% menthol (photograph B), and 1 wt% PG + 5 wt% menthol (photograph C).

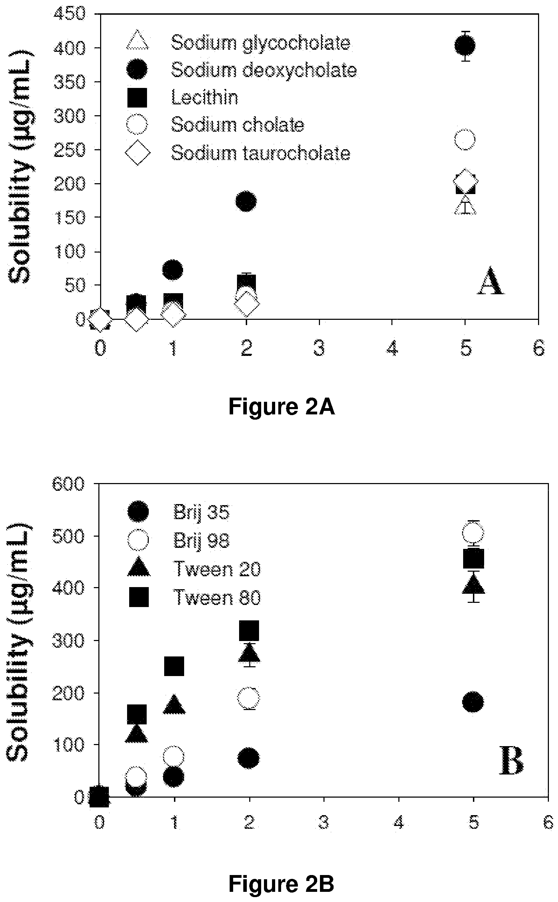

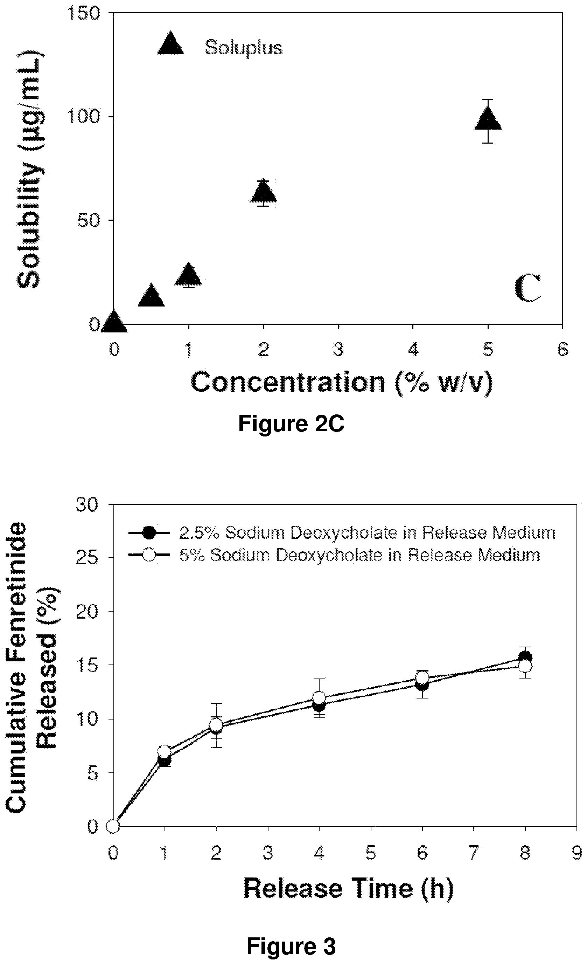

**Figures 2A****-C:** Graphs showing solubilization of fenretinide in simulated saliva (buffer, pH 6.8). Effect of addition of bile salt/lecithin **(****Figure 2A****),** surfactant **(****Figure 2B****),** and hydrophilic polymer **(****Figure 2C****)** on the solubility of fenretinide in simulated saliva. Solubility of fenretinide in simulated saliva in the presence of 0.5, 1, 2, and 5 %w/v solubilizers at 37°C. Values represent mean ± SE, *n* = 3.
**Figure 3****:** Graph showing effect of addition of sodium deoxycholate in simulated saliva (pH 6.8) on cumulative release of fenretinide from Eudragit® RS-PO film. Drug loading was 5wt%.

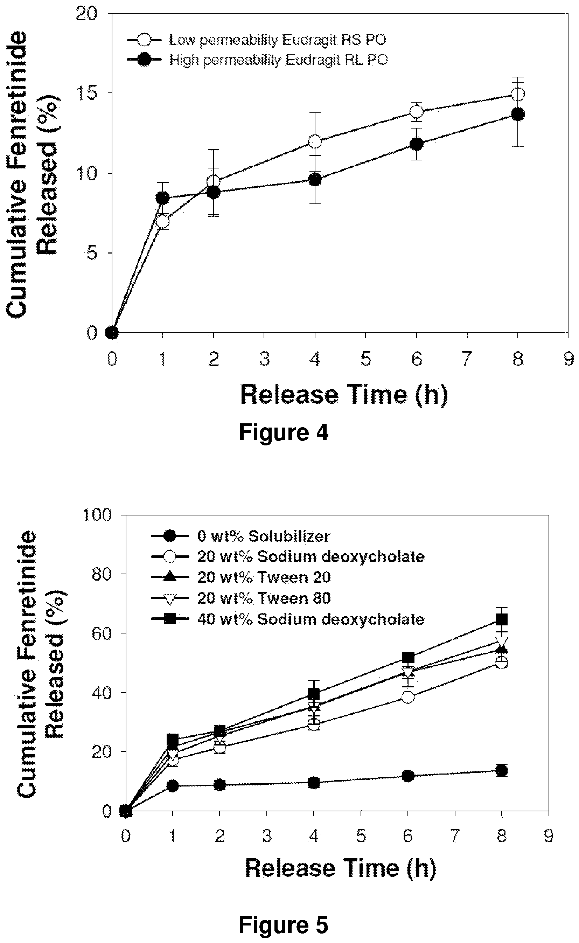

**Figure 4****:** Graph showing effect of polymer matrix permeability on cumulative release of fenretinide from Eudragit® film. Drug loading was 5wt%. Release study was conducted in simulated saliva (buffer, pH 6.8) containing 5 %w/v sodium deoxycholate at 37°C. Values represent mean ± SE, *n* = 3.
**Figure 5****:** Graph showing effect of co-encapsulation of solubilizer on cumulative release of fenretinide from Eudragit® RL PO film. Drug loading was 5wt%. Release study was conducted in simulated saliva (buffer, pH 6.8) containing 5 %w/v sodium deoxycholate at 37°C. Values represent mean ± SE, *n* = 3.

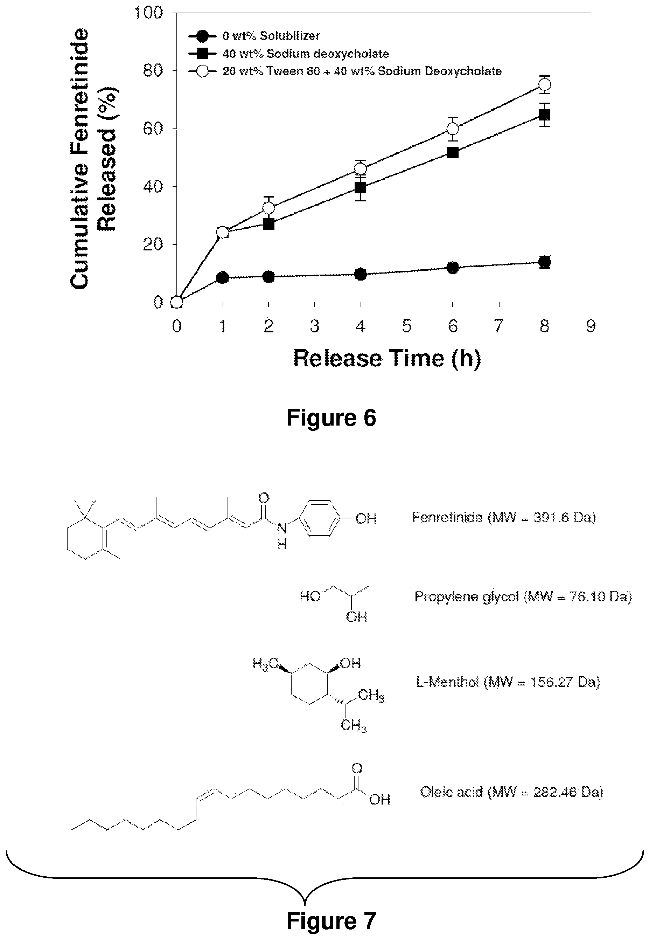

**Figure 6****:** Graph showing effect of co-encapsulation of mixed solubilizers on cumulative release of fenretinide from Eudragit® RL PO film. Drug loading was 5wt%. Release study was conducted in simulated saliva (buffer, pH 6.8) containing 5 %w/v sodium deoxycholate at 37°C. Values represent mean ± SE, *n* = 3.
**Figure 7****:** Chemical structures of fenretinide and the chemical permeation enhancers propylene glycol, L-menthol, and oleic acid.

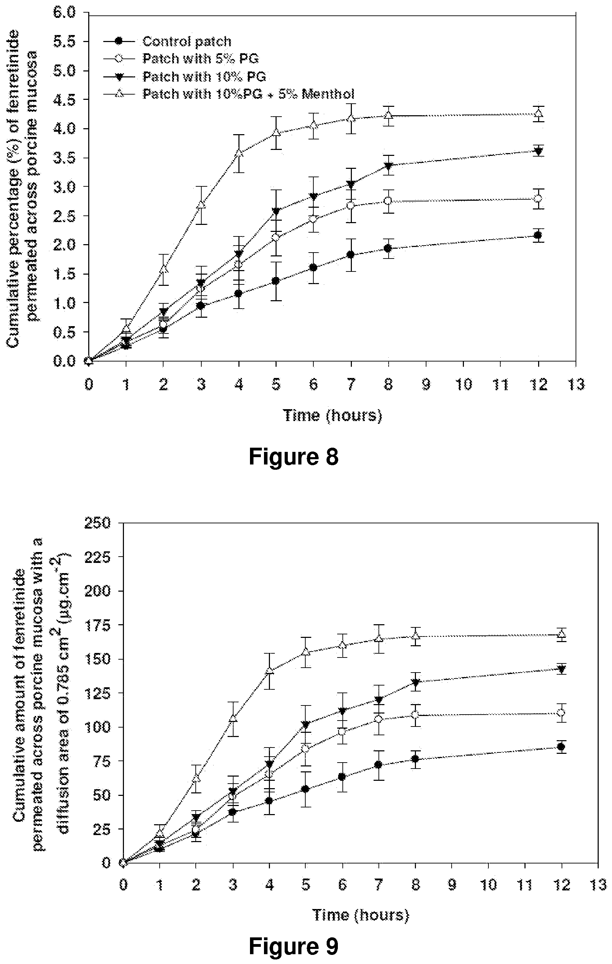

**Figure 8****:** Graph showing cumulative percentage versus time profiles of fenretinide permeated across porcine buccal mucosa from patches with/without propylene glycol (mean ± SD, n =3).
**Figure 9****:** Graph showing cumulative amount versus time profiles of fenretinide permeated across porcine buccal mucosa from patches with/without propylene glycol (mean ± SD, n =3).

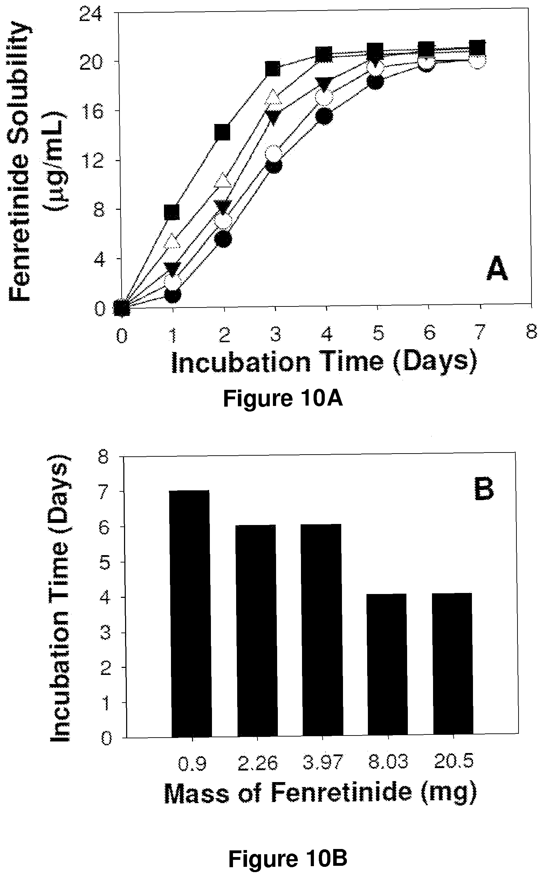

**Figures 10A-10B****:** Graph showing solubilization of fenretinide in bovine serum. The effect of quantity (0.9 (●), 2.26 (○), 3.97 (▼), 8.03 (Δ), and 20.05 (■)) of fenretinide added in 15-mL bovine serum and incubation time on the solubility of fenretinide **(****Figure 10A**) and the relationship between the quantity of fenretinide added and time required to reach equilibration **(****Figure 10B**). Solubility study was conducted at 37°C under the protection from light.

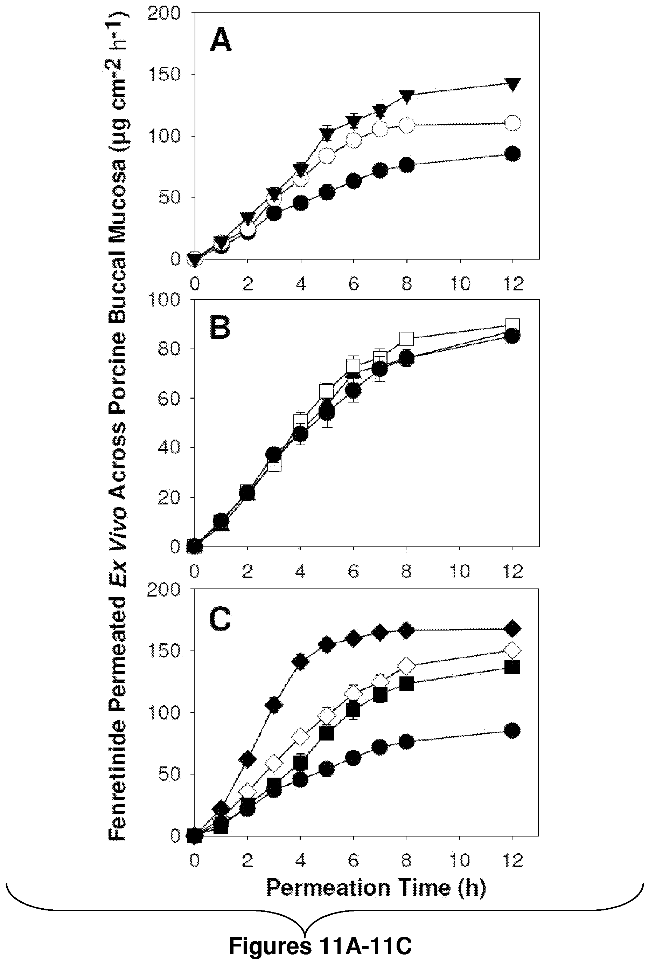

**Figures 11A-11C****:** Graph showing co-incorporation of propylene glycol (PG) or PG + menthol in fenretinide/Eudragit® RL PO patches significantly enhance fenretinide permeation across porcine buccal mucosa. The effect of co-incorporation of 0 (●), 5 (○) and 10 (▼) wt% PG **(****Figure 11A****);** 5 (▲) and 10 (▲) wt% menthol **(****Figure 11B****),** and 1 wt% PG + 5 wt% menthol (▲), 2.5 wt% PG + 5 wt% menthol (◊) and 10 wt% PG + 5 wt% menthol (◆) **(****Figure 11C****)** in patches on *ex vivo* permeation of fenretinide across porcine buccal mucosa. *Ex vivo* permeation studies were conducted using side-by-side flow-through diffusion cells at 37°C. Permeation enhancer-free patch comprised of 5 wt% fenretinide, 20 wt% Tween® 80, and 40 wt% sodium deoxycholate. Symbols represent mean ± SE, n = 5.

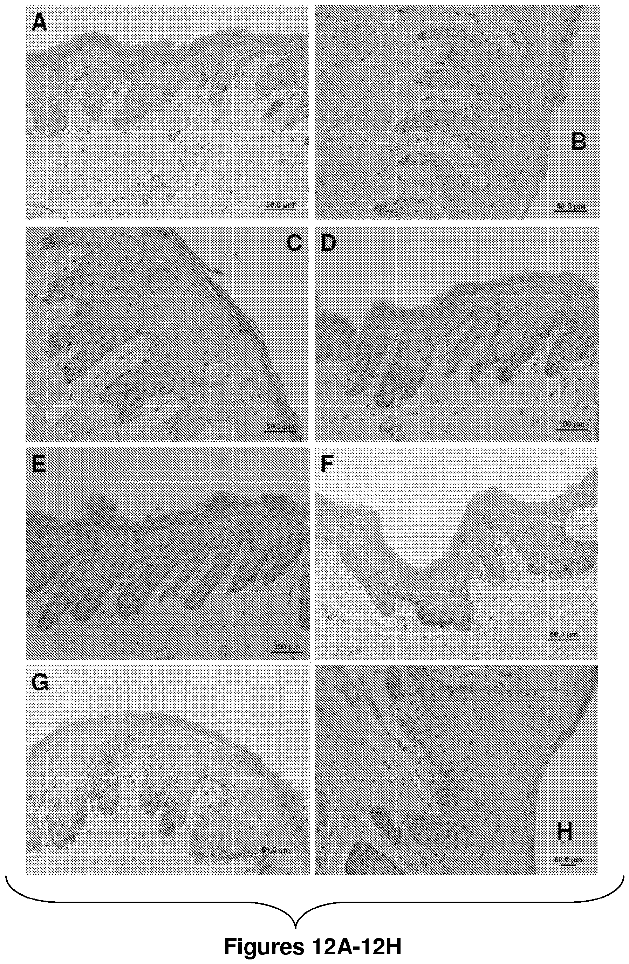

**Figures 12A-12H****:** Photographs showing histological examination of porcine buccal tissue before and after fenretinide buccal permeation enhancement with permeation enhancers loaded mucoadhesive patches. The effect of co-incorporation of 0 wt% **(****Figure 12A****),** 5 wt% **(****Figure 12B****)** or 10 wt% **(****Figure 12C****)** propylene glycol (PG); 5 wt% **(****Figure 12D****)** or 10 wt% **(****Figure 12E****)** wt% menthol; and, 1 wt% PG + 5 wt% menthol **(****Figure 12F****),** or 2.5 wt% PG + 5 wt% menthol **(****Figure 12G****),** or 10 wt% PG + 5 wt% menthol **(****Figure 12H****)** in fenretinide/Eudragit RL PO mucoadhesive patches on histological changes of porcine buccal tissue. As shown in these photomicrographs, patch application (with or without permeation enhancers) did not dramatically perturb porcine buccal mucosa. All sections demonstrate a preserved basement membrane and basal cell layer, an intact stratified squamous surface epithelium with an overlying parakeratotic layer. Notably, no evidence of changes consistent with extensive epithelial damage e.g., hydropic degeneration of the basal cell layer or acantholysis were observed in multiple sections. The evidence of increased intracellular and intercellular edema observed in epithelia exposed to increased levels PG (**Figure 12C** and **Figure 12H****)** likely reflects diffusion of PG into subject keratinocytes as well as the intercellular spaces. Images were taken by a light microscope. Permeation enhancer-free patch comprised of 5 wt% fenretinide, 20 wt% Tween® 80, and 40 wt% sodium deoxycholate. Release Time (h).

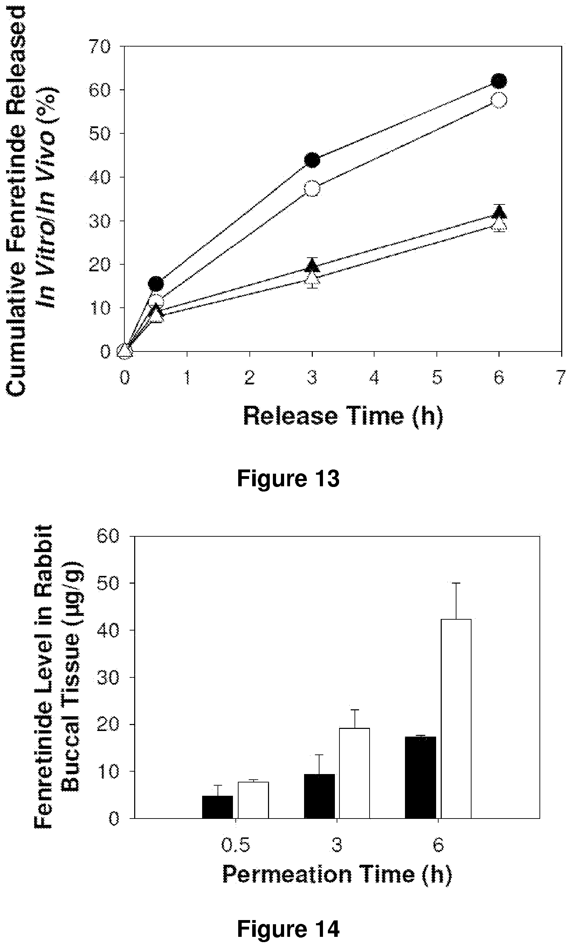

**Figure 13****:** Graph showing *in vitro* and *in vivo* release characteristics of permeation enhancers-free and permeation enhancers-loaded fenretinide/Eudragit® RL PO mucoadhesive patches. Cumulative amount of fenretinide released *in vitro*/*in vivo* from permeation enhancers-free (○: *in vitro;* Δ: *in vivo*) and permeation enhancers (2.5 wt% propylene glycol + 5 wt% menthol)-loaded (●: *in vitro;* ▲*: in vivo*) patches as a function of time. *In vitro* and *in vivo* release studies were conducted in simulated saliva containing 5% w/v sodium deoxycholate (pH 6.8) at 37°C and rabbits, respectively. Permeation enhancer-free patch comprised of 5 wt% fenretinide, 20 wt% Tween® 80, and 40 wt% sodium deoxycholate. Symbols represent mean ± SE, n = 4 (*in vitro*) or 6 (*in vivo*).
**Figure 14****:** Graph showing co-incorporation of Permeation enhancers (2.5 wt% propylene glycol + 5 wt% menthol) in fenretinide/Eudragit® RL PO patch enhances *in vivo* buccal mucosal permeation of fenretinide. Tissue levels of fenretinide as a function of buccal administration time of permeation enhancer-free (filled bars) and permeation enhancers-loaded (open bars) patches in rabbits. Permeation enhancer-free patch comprised of 5 wt% fenretinide, 20 wt% Tween® 80, and 40 wt% sodium deoxycholate. Bars represent mean ± SE, n = 6).

### DETAILED DESCRIPTION OF THE PREFERRED EMBODIMENT

Throughout this disclosure, various publications, patents and published patent specifications are referenced by an identifying citation. The disclosures of these publications, patents and published patent specifications assist in describing the state of the art to which this invention pertains.

In describing and claiming the present invention, the following terminology will be used in accordance with the definitions set out below.

As used in the specification and claims, the singular form "a," "an," and "the" include plural references unless the context clearly dictates otherwise. For example, the term "a cell" includes a plurality of cells, including mixtures thereof.

As used herein, the terms "may," "optionally," and "may optionally" are used interchangeably and are meant to include cases in which the condition occurs as well as cases in which the condition does not occur. Thus, for example, the statement that a formulation "may include an excipient" is meant to include cases in which the formulation includes an excipient as well as cases in which the formulation does not include an excipient.

As used here, the terms "beneficial agent" and "active agent" are used interchangeably herein to refer to a chemical compound or composition that has a beneficial biological effect. Beneficial biological effects include both therapeutic effects, i.e., treatment of a disorder or other undesirable physiological condition, and prophylactic effects, i.e., prevention of a disorder or other undesirable physiological condition (e.g., cancer). The terms also encompass pharmaceutically acceptable, pharmacologically active derivatives of beneficial agents specifically mentioned herein, including, but not limited to, salts, esters, amides, prodrugs, active metabolites, isomers, fragments, analogs, and the like. When the terms "beneficial agent" or "active agent" are used, then, or when a particular agent is specifically identified, it is to be understood that the term includes the agent per se as well as pharmaceutically acceptable, pharmacologically active salts, esters, amides, prodrugs, conjugates, active metabolites, isomers, fragments, analogs, etc.

As used herein, the terms "treating" or "treatment" of a subject includes the administration of a drug to a subject with the purpose of preventing, curing, healing, alleviating, relieving, altering, remedying, ameliorating, improving, stabilizing or affecting a disease or disorder, or a symptom of a disease or disorder. The terms "treating" and "treatment" can also refer to reduction in severity and/or frequency of symptoms, elimination of symptoms and/or underlying cause, prevention of the occurrence of symptoms and/or their underlying cause, and improvement or remediation of damage.

As used herein, the term "preventing" a disorder or unwanted physiological event in a subject refers specifically to the prevention of the occurrence of symptoms and/or their underlying cause, wherein the subject may or may not exhibit heightened susceptibility to the disorder or event.

By the term "effective amount" of a therapeutic agent is meant a nontoxic but sufficient amount of a beneficial agent to provide the desired effect. The amount of beneficial agent that is "effective" will vary from subject to subject, depending on the age and general condition of the subject, the particular beneficial agent or agents, and the like. Thus, it is not always possible to specify an exact "effective amount." However, an appropriate "effective" amount in any subject case may be determined by one of ordinary skill in the art using routine experimentation. Also, as used herein, and unless specifically stated otherwise, an "effective amount" of a beneficial can also refer to an amount covering both therapeutically effective amounts and prophylactically effective amounts.

An "effective amount" of a drug necessary to achieve a therapeutic effect may vary according to factors such as the age, sex, and weight of the subject. Dosage regimens can be adjusted to provide the optimum therapeutic response. For example, several divided doses may be administered daily or the dose may be proportionally reduced as indicated by the exigencies of the therapeutic situation.

As used herein, a "therapeutically effective amount" of a therapeutic agent refers to an amount that is effective to achieve a desired therapeutic result, and a "prophylactically effective amount" of a therapeutic agent refers to an amount that is effective to prevent an unwanted physiological condition. Therapeutically effective and prophylactically effective amounts of a given therapeutic agent will typically vary with respect to factors such as the type and severity of the disorder or disease being treated and the age, gender, and weight of the subject.

The term "therapeutically effective amount" can also refer to an amount of a therapeutic agent, or a rate of delivery of a therapeutic agent (e.g., amount over time), effective to facilitate a desired therapeutic effect, such as pain relief. The precise desired therapeutic effect will vary according to the condition to be treated, the tolerance of the subject, the drug and/or drug formulation to be administered (e.g., the potency of the therapeutic agent (drug), the concentration of drug in the formulation, and the like), and a variety of other factors that are appreciated by those of ordinary skill in the art.

As used herein, the term "pharmaceutically acceptable" component can refer to a component that is not biologically or otherwise undesirable, i.e., the component may be incorporated into a pharmaceutical formulation (e.g. a formulation according to the present invention) and administered to a subject as described herein without causing any significant undesirable biological effects or interacting in a deleterious manner with any of the other components of the formulation in which it is contained. When the term "pharmaceutically acceptable" is used to refer to an excipient, it is generally implied that the component has met the required standards of toxicological and manufacturing testing or that it is included on the Inactive Ingredient Guide prepared by the U.S. Food and Drug Administration.

Also, as used herein, the term "pharmacologically active" (or simply "active"), as in a "pharmacologically active" derivative or analog, can refer to a derivative or analog (e.g., a salt, ester, amide, conjugate, metabolite, isomer, fragment, etc.) having the same type of pharmacological activity as the parent compound and approximately equivalent in degree.

As used herein, the term "mixture" can include solutions in which the components of the mixture are completely miscible, as well as suspensions and emulsions, in which the components of the mixture are not completely miscible.

As used herein, the term "subject" can refer to living organisms such as mammals, including, but not limited to humans, livestock, dogs, cats, and other mammals. Administration of the therapeutic agents can be carried out at dosages and for periods of time effective for treatment of a subject. The subject may be a human. The pharmacokinetic profiles of the systems disclosed herein may be similar for male and female subjects.

As used herein, the term "controlled drug delivery" refers to release or administration of a drug from a given dosage form in a controlled fashion in order to achieve the desired pharmacokinetic profile *in vivo.* An aspect of "controlled" drug delivery is the ability to manipulate the formulation and/or dosage form in order to establish the desired kinetics of drug release.

As used herein, the term "sustained drug delivery" refers to release or administration of a therapeutic agent from a source (e.g., a drug formulation) in a sustained fashion over a protracted yet specific period of time, which may extend from several minutes to a few hours, days, weeks or months. The term "sustained" can refer to delivery of consistent levels of the therapeutic agent over a time period ranging from a few minutes to a day, with a profile characterized by the absence of an immediate release phase, such as the one obtained from intravenous administration.

The present invention is based, at least in part, on the discovery that transmucosal uptake of fenretinide can be enhanced by employing permeation enhancing agents. Such permeation enhancing agents are advantageous, e.g., because the absolute bioavailability of the therapeutic agent contained therein is enhanced, while also providing a desired delivery time period for the therapeutic agent. Additionally, less therapeutic agent is needed in the system to deliver a therapeutic effect versus systems of the prior art.

In particular, there has been a major challenge in developing an effective mucoadhesive system is to achieve continuous and complete drug-release from the system.

Described herein is a mucoadhesive system which now provides a continuous and near complete drug-release. The mucoadhesive system, as now described herein provides an improved technology where systemic administration using the mucoadhesive system provides therapeutic levels to the mouth without inducing significant side effects.

The mucoadhesive system-based approach provides a targeted delivery of therapeutic levels of fenretinide at a treatment site without induction of deleterious systemic effects.

The mucoadhesive system described herein overcomes the issues associated with the efficacy of delivery systems for various hydrophobic drugs.

The term "transmucosal," as used herein, refers to any route of administration via a mucosal membrane. Examples include, but are not limited to, buccal, sublingual, nasal, vaginal, and rectal. The administration may be buccal. The administration may be sublingual. As used herein, the term "direct transmucosal" refers to mucosal administration via the oral mucosa, e.g. buccal and/or sublingual.

The term "buccal patch" or "film" typically refers to a flexible film that adheres to the oral mucosa and delivers the therapeutic agent. Such films can be either quick dissolving or dispersing films releasing the therapeutic agent immediately or can be films having mucoadhesive properties with the therapeutic being released over a period of time. These patches or films are typically prepared by mixing the ingredients, heating, extruding, drying and then sizing the sheets to deliver the exact amounts of medications.

The present invention provides a formulation for transmucosal administration that is easy to manufacture, exhibits good stability and allows for flexibility of formulation.

The present invention provides a formulation for transmucosal administration that allows for precise control over the dose administered and the effect obtained.

The present invention provides a formulation for transmucosal administration that is simple, convenient to administer, easy to handle and promotes high patient acceptance and compliance.

The mucoadhesive system may comprise the use of effective solubilizers that enhances the release of the therapeutic agent from the patch.

The solubilizer and the permeation enhancer may be co-incorporated with the therapeutic agent in a drug layer film, as schematically illustrated in **Figures 1A-1C****,** and as further described below. The solubilizer enhances the release of the therapeutic agent from the drug film layer, while the permeation enhancer improves the permeation of the therapeutic agent across the mucosa. It is to be noted, however, the formulation may be a "single" formulation comprised of the solubilizer and the therapeutic agent.

Provided herein is a controlled release retinide mucoadhesive system.

Provided herein are formulations of mucoadhesive system for effective controlled release of a therapeutic composition.

Methods for transmucosal administration of a therapeutic agent where a transmucosal patch is applied to a mucosal membrane and kept in contact with it for a therapeutically effective period of time are also provided. When the desired therapeutic effect has been achieved, the patch can be optionally removed.

The formulation can be delivered by the various transmucosal routes. In particular, the formulation can be delivered via the buccal mucosa. The buccal mucosa is readily accessible and provides a desired wide area of smooth muscle for application of a patch. In addition, absorption through the buccal mucosa delivers the therapeutic agent directly into the systemic circulation through the internal jugular vein, thus bypassing the hepatic metabolic system. Also, the buccal mucosa tends to have low enzymatic activity, and delivery of the therapeutic agent through the buccal mucosa thus avoids degradation in the gastric and intestinal fluids.

The formulation may be suitable for both immediate delivery and time-controlled delivery of the therapeutic agent though the buccal mucosa.

### Design of controlled release mucoadhesive system

In the embodiment illustrated in **Figures 1A-1C****,** a "patch" or mucoadhesive delivery system 10 generally includes a backing layer 12, an adhesive layer 14, and drug-release layer 20. As disclosed herein, the terms "delivery system" and "patch" can be used interchangeably. The patch 10 is designed to be applied to a mucosal membrane, and is used to deliver a therapeutic agent through transmucosal administration. The delivery patch 10 can be of any shape and size as desired.

Each mucoadhesive layer of the system perform specific role and contribute towards effective controlled delivery of one or more therapeutic agents (i.e., drugs). The backing layer 12 (which is insoluble in saliva, water, and the like) prevents the drug loss/release from a rear surface of the drug-release layer 20, thereby providing unidirectional drug-release. The adhesive layer 14 provides strong mucoadhesion with mucosal surfaces. The drug-release layer 20 can provide a substantially continuous and complete drug delivery.

As described in the Examples herein, the backing layer 12 can be comprised of a Tegaderm® dressing film; the adhesive layer 14 can be comprised of one or more mucoadhesive polymers; and, the drug-release layer 20 can be comprised of a formulation comprised of a therapeutic agent, a for example, a Eudragit® polymer + drug solubilizer).

Referring to Figure 1C, there is shown a schematic cross-sectional view of patch 10. The adhesive layer 12 can have any suitable general overall configuration. As shown in Figure 1C, the adhesive layer 12 can define a recess 18. It is to be understood that the depth of the recess can be readily determined by those skilled in the art. The adhesive layer 18 has an outer adhesive surface 19 that is exposed when a protective layer (not shown) is removed when the patch is ready for placement in a subject.

As shown in **Figures 1A-1C****,** the drug-release layer 20 can be configured to fit into the recess 18 such that an outer drug-release surface 22 of the drug-release layer 20 is exposed to the mucosa (not shown) when in use. The remaining sides and inner surfaces of the drug-release layer 20 can be substantially surrounded by the adhesive layer 14. Both the adhesive layer 14 and the drug-release layer 22 together can form a unitary construction. In such construction, the outer drug-release surface 22 of the drug-release layer 20 is substantially planar with a plane defined by the outer adhesive surface 19 of the adhesive layer 14 such that, when the patch 10 adheres to a mucosal membrane, both the outer drug-release surface 22 and the outer adhesive layer are in contact with the mucosa (not shown).

The dimensions of both the adhesive layer 14 and the drug-release layer 20 are chosen as per the therapeutic agent to be administered and its dosage. The ratio of lengths of both (i.e., diameters in case of a circular recess) can be selected such that the adhesive surface 19 has sufficient area to enable adequate adhesion of the patch 10 to the mucosa. The thickness of the patch 10 is kept at a minimum possible, to ensure least possible foreign body sensation and better patient compliance and mouth-feel. For example, the thickness can range from about 0.5 mm to about 5 mm. The patch may be of a circular shape, but alternative shapes of the patch 10 can be easily manufactured. Non-limiting examples of other shapes include oval, ellipsoidal, capsule shape, and the like. The shapes may lack sharp edges such that the patch is less likely to raise concerns such as mechanical instability and irritation during use. However, it is to be understood that other modifications to the shape, such as making various geometric shapes or making both the compartments of different shapes are obvious to a person skilled in the art and are contemplated as part of this disclosure.

The drug-release layer 20 contains a formulation comprising one or more therapeutic agent/agents, and optionally excipients. The therapeutic agent can be present in the range of about 0.1 to about 99% w/w, preferably about 1 to about 90% w/w of the patch, depending on its dose and formulation factors.

### Formulations and Therapeutic Formulations

The terms "therapeutic formulation/s" and 'formulation/s" can be used interchangeably herein. According to one aspect of the present invention, the formulation includes: at least one mucoadhesive material; at least an effective amount of fenretinide or a pharmaceutically acceptable salt thereof; and, at least one transmucosal permeation enhancer agent comprising propylene glycol and menthol; and, at least one solubilizer agent.

The present invention further provides the formulation of the present invention for use in a method of treatment and prophylaxis of a disease or disorder, said use comprising administering to a subject in need of such treatment.

The formulations can be provided as: a gel, a rinse, and two locally injectable delivery formulations: one that can be delivered from a poly-lactide-co-glycolide, and another that can be delivered as a gel that undergoes hybridization after injection and achieving body temperature.

### Mucoadhesive Materials

The formulation includes one or more mucoadhesive materials, optionally in combination with suitable excipients. In certain embodiments, the mucoadhesive material is present in the range of about 1% to about 99% w/w, preferably about 5 to about 95% w/w of the formulation.

Useful examples of mucoadhesive materials include polymers of acrylic acid esters, acrylic acid copolymers, vinyl polymers, vinyl copolymers, polymers of vinyl alcohols, carboxy vinyl polymers and copolymers, vinyl esters, alkoxy polymers, polyethylene oxide polymers, polyethers, and mixtures thereof.

In the examples herein the mucoadhesive materials comprised a methacrylate copolymer. One non-limiting example is commercially available under the tradename Eudragit ®, which is a copolymer of ethyl acrylate, methyl methacrylate and a low content of methacrylic acid ester with quaternary ammonium groups. The ammonium groups are present as salts and make the polymers permeable. The Chemical/IUPAC name is poly(ethyl acrylate-co-methyl methacrylate-co-trimethylammonioethyl methacrylate chloride) 1:2:0.1; INCI name: Acrylates/ Ammonium Methacrylate Copolymers.

The mucoadhesive material may be admixed with other materials; for example, the mucoadhesive materials may be admixed with optional excipients, such as binders, coloring agents; diluents, enzyme inhibitors, fillers, flavoring agents, lubricants, stabilizers, sweetening agents, and the like.

### Therapeutic Agents

The therapeutic formulation described herein is especially useful in the treatment of subjects with precancerous oral epithelial lesions.

The therapeutic agent disclosed herein is a hydrophobic composition, such as synthetic Vitamin A compositions, such as retinide compositions. The mucoadhesive system disclosed herein may include a formulation that is especially useful for the delivery of retinide compositions. The retinide composition used in the present invention is a synthetic retinoid, fenretinide. Fenretinide (4-hydroxy(phenyl)retinamide) is a highly lipophilic drug and has a log P of 8.03, which results in minimal buccal mucosal uptake and permeation. The chemical structures of the drug and permeation enhancers are shown in **Figure 7****.**

In the past, however, the achievement of desired antitumor activity using fenretinide has been limited by low bioavailability (due to low membrane permeability) and rapid elimination from the body respectively after oral and intravenous administration of fenretinide. Therefore, multiple dosing of fenretinide is required to achieve therapeutic drug level in the blood and hence potent oral cancer chemoprevention. The local delivery from hydrogel-based mucoadhesive system provides therapeutic fenretinide level directly at the treatment site, thereby improving the therapeutic efficacy of fenretinide in cancer chemoprevention. However, fenretinide is a highly hydrophobic drug with very low water solubility (below HPLC detection limit). Though fenretinide possesses both desirable epithelial differentiation and apoptotic-inducing capabilities, its previous clinical use has been limited to oral systemic administration.

### Transmucosal Permeation Agents

The transmucosal permeation enhancing agents described in more detail herein provide an enhanced delivery profile and more efficient delivery of the therapeutic agent. Additional advantages of the transmucosal permeation enhancing agents are also described herein. The transmucosal permeation enhancer agents disclosed herein include propylene glycol (PG) and terpenoids or terpenes (such as menthol, D-limonene, geraniol, nerolidol) and mixtures thereof.

The transmucosal permeation enhancer agent used in the present invention comprises propylene glycol (PG) and menthol. In one embodiment, the transmucosal permeation enhancer agent comprises from about 1 wt% to about 2.5 wt% PG, and from 1 wt % to about 5 wt% menthol.

In another embodiment, the transmucosal permeation enhancer agent comprises from about 1 wt % to about 2.5 wt% PG, and about 5 wt% menthol.

In another embodiment, the transmucosal permeation enhancer agent comprises about 1 wt% PG and about 5 wt% menthol.

In yet another embodiment, the transmucosal permeation enhancer agent comprises about 2.5 wt% PG and about 5 wt% menthol.

### Solubilizers

In certain embodiment, the formulation includes an effective amount on one or more solubilizers to facilitate continuous *in vitro* and *in vivo* release from the mucoadhesive material. To maintain a desired sink condition in the release/receiver chamber medium, appropriate quantity of suitable solubilizing agent can be incorporated. In this Example, the optimal quantity of nonionic surfactant in the release media was selected by matching the drug solubility to that in bovine serum.

Non-limiting examples of solubilizers include, deoxycholic acid, polyoxy-ethylene-sorbitan higher fatty acid esters (e.g., Tween®80).

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various usages and conditions. The following examples are intended to illustrate certain preferred embodiments of the invention and should not be interpreted to limit the scope of the invention as defined in the claims, unless so specified.

### EXAMPLE 1

### Preparation of hydrogel-based controlled release fenretinide mucoadhesive system:

### Preparation of an adhesive layer

An adhesive layer based on the blend of hydroxypropyl methylcellulose (HPMC 4KM) and polycarbophil (PC) at a weight ratio of 3:1 was prepared by a casting method. Briefly, 1.5% polymer solution was prepared in ddH₂O containing required amount (20 wt% based on polymer mass) of propylene glycol by stirring the polymer/water mixture overnight. About 50 mL of polymer solution was then casted onto glass petri dish (150 × 20 mm) and incubated at 50 °C for 48 h. Then, the polymer film was cut into required size and stored in a desiccator at room temperature until further use.

### Preparation of drug-release (fenretinide) layer/film

Preparation of fenretinide films was performed under the protection from light. A desired quantity of solubilizer (Tween® 80 and sodium deoxycholate), permeation enhancers (1, 2.5, 5, and 10 wt%) and mucoadhesive material, Eudragit® RL PO, were weighed in 15 mL polypropylene tubes to which 8 mL of a 50:50 (v/v) acetone-ethanol mixture was added. The quantity of plasticizer or solubilizer added was calculated based on the mass of polymer. The resulting mixture was vortexed until all ingredients were dissolved. The required quantity (5 wt% based on the total mass of polymer + excipients) of fenretinide was then added to above prepared polymer-solubilizer or polymer-solubilizer-permeation enhancer(s) solution, vortexed again, and the volume was adjusted to 10 mL with the same solvent mixture. Five milliliter of fenretinide-polymer solution was added onto Teflon (Scientific Commodities, Inc., Lake Havasu City, AZ, USA) overlaid glass petri dish (60 × 15 mm) and incubated at 38°C for 48 h. After sufficient drying, fenretinide loaded polymer film was cut into required size (7 mm diameter), packed in aluminum foil, and stored in a desiccator at -20 °C until further use.

### Assembly of oral mucoadhesive patches of fenretinide

An annular adhesive layer with 11 (outer diameter) and 7 (inner diameter) mm dimensions were formed by cutting the film with 11 and 7 mm cork borers, respectively. The adhesive layer was then placed onto adhesive side of the Tegaderm™ film (backing layer), followed by insertion of previously cut 7 mm fenretinide/Eudragit® layer into open region of adhesive layer to obtain oral mucoadhesive patch of fenretinide. **Figure ID** shows the physical appearance of the fenretinide/Eudragit® (drug-release) layer loaded with 5 wt% menthol (photograph A), 10% menthol (photograph B), and 1 wt% PG + 5 wt% menthol (photograph C).

### Effect of Co-incorporation of Menthol on Fenretinide/Eudragit® RL PO Film Morphology

Fenretinide/Eudragit® RL PO (drug-release) films without menthol exhibited good film forming and physical appearance. Fenretinide/Eudragit® RL PO films loaded with 5% or 10% menthol did not exhibit good film forming and physical appearance (see photograph A and photograph B in **Figure 1D**). It appeared that that phase separation occurred during the film formation due to formation precipitation and/or aggregation of menthol. As shown in photograph C in **Figure 1D**, the addition of 1% PG as a co-solvent facilitated desirable film formation.

### Solubilization of fenretinide in simulated saliva with variety of solubilizers

The extent of solubility enhancement of fenretinide by numerous solubilizers (bile salts, surfactants, hydrophilic polymers, and co-solvents) was studied by determining the solubility in simulated saliva in the presence of 0.5, 1, 2, and 5 %w/v solubilizers. Briefly, an excess amount of fenretinide was added into separate amber color ampoules containing 1-mL 0.5, 1, 2, and 5 %w/v solutions of solubilizers (prepared using N₂-purged simulated saliva) and sealed under vacuum in order remove the oxygen from the head-space. The ampoules were then placed in an incubator maintained at 37°C and shaken at 240 RPM for 72 h (this duration was determined to be sufficient to reach the equilibrium). After 72 h, the ampoules were broken, mixture was passed through 0.45 µm PVDF filter units (Millipore, USA), diluted suitably with respective solubilizer solution, and the amount of fenretinide solubilized in simulated saliva was determined by HPLC.

### RESULTS for Example 1

**Figures 2A-2C** show graphs illustrating the solubilization of fenretinide in simulated saliva (buffer, pH 6.8). Effect of addition of bile salt/lecithin **(****Figure 2A****),** surfactant **(****Figure 2B****),** and hydrophilic polymer **(****Figure 2C****)** on the solubility of fenretinide in simulated saliva. Solubility of fenretinide in simulated saliva in the presence of 0.5, 1, 2, and 5 %w/v solubilizers at 37°C. Values represent mean ± SE, *n* = 3.

**Table 1 below shows examples of formulations of hydrogel-based controlled release fenretinide mucoadhesive system that were evaluated.**

| **Table 1: Compositions of hydrogel-based controlled release fenretinide mucoadhesive system** | | |
|---|---|---|
| **Adhesive layer** | | |
| 1) | HPMC 4KM | |
| 2) | Polycarbophil | |
| 3) | Propylene glycol | |

| **Drug (fenretinide)-release layer** | | |
|---|---|---|
| ***A)*** | ***Solubilizer-free fenretinide layer*** | |
| | I) | Fenretinide |
| | 2) | Eudragit® polymer |
| | 3) | Triacetyl citrate |
| | | |

| ***B)*** | ***Solubilizer-loaded fenretinide layer*** | |
|---|---|---|
| | 1) | Fenretinide |
| | 2) | Eudragit® polymer |
| | 3) | Solubilizer |
| | | Tween® 20 or Tween® 80 or Brij 98 or Sodium deoxycholate |

The fenretinide loading efficiency of Eudragit® RS PO/RL PO films is shown in **Table 2** below.

| **Table 2:** Evaluation of microencapsulation of fenretinide in solubilizer-free and solubilizers loaded Eudragit® RS PO/RL-PO films | | | |
|---|---|---|---|
| **Formulation** | **Fenretinide Loading** | | **Loading Efficiency** (%)* |
| | **Theoretical^{a}** (wt%) | **Actual** (wt%)* | |
| Eudragit® RS-PO | 5.0 | 4.5 ± 0.1 | 90.0 ± 1.2 |
| Solubilizer-free Eudragit® RL-PO | | | |
| | 5.0 | 4.6 ± 0.1 | 92.1 ± 1.0 |
| | 10.0 | 9.2 ± 0.2 | 92.0 ± 2.0 |
| Solubilizers Loaded Eudragit® RL-PO | | | |
| 20 wt% Tween® 20 | 5.6 | 5.4 ± 01 | 96.3 ± 1.5 |
| 20 wt% Tween® 80 | 5.1 | 4.9 ± 0.2 | 95.1 ± 1.6 |
| 20 wt% Brij 98 | 5.2 | 4.8 ± 0.1 | 92.3 ± 1.5 |
| 20 wt% Sodium deoxycholate | 5.6 | 5.5 ± 0.2 | 97.0 ± 1.0 |
| 40 wt% Sodium deoxycholate | 5.0 | 4.6 ± 0.1 | 91.4 ± 1.0 |
| 20 wt% Tween® 80 + 40 wt% Sodium deoxycholate | 4.9 | 4.5 ± 0.2 | 92.0 ± 2.1 |
| Mean ± SE, n=3; "Based on polymer + excipients weight | | | |

### Identification of suitable release medium to maintain sink condition during in vitro drug-release

The drug is insoluble in simulated saliva (pH 6.8). Therefore, it is not possible to maintain the sink condition in simulated saliva during the *in vitro* drug-release studies. To maintain the sink condition, 2.5 and 5 % (w/v) sodium deoxycholate was added into the simulated saliva. The solubilizer used to maintain sink condition may be inert and does not change the drug release characteristics of film. To understand phenomenon, *in vitro* release study of fenretinide from Eudragit® films in simulated saliva containing 2.5 and 5 % (w/v) sodium deoxycholate was performed.

**Figure 3** illustrates the effect of addition of sodium deoxycholate in simulated saliva (pH 6.8) on cumulative release of fenretinide from Eudragit® RS-PO film. Drug loading was 5wt%. The addition of different fraction of sodium deoxycholate did not change the drug release characteristics of polymer film, suggesting that the solubilizer is inert and can be used to maintain sink condition in the simulated saliva. Further release studies were conducted in simulated saliva containing 5 %w/v sodium deoxycholate.

### Effect of polymer matrix permeability on in vitro release of fenretinide from Eudragit® films

**Figure 4** illustrates the effect of polymer matrix permeability on cumulative release of fenretinide from Eudragit® film. Drug loading was 5wt%. Release study was conducted in simulated saliva (buffer, pH 6.8) containing 5 %w/v sodium deoxycholate at 37 °C. Values represent mean ± SE, *n* = 3.

### Effect of co-encapsulation of solubilizer on in vitro release of fenretinide from Eudragit® films

**Figure 5** illustrates the effect of co-encapsulation of solubilizer on cumulative release of fenretinide from Eudragit® RL PO film. Drug loading was 5wt%. Release study was conducted in simulated saliva (buffer, pH 6.8) containing 5 %w/v sodium deoxycholate at 37°C. Values represent mean ± SE, *n* = 3.

**Figure** 6 illustrates the effect of co-encapsulation of mixed solubilizers on cumulative release of fenretinide from Eudragit® RL PO film. Drug loading was 5wt%. Release study was conducted in simulated saliva (buffer, pH 6.8) containing 5 %w/v sodium deoxycholate at 37°C. Values represent mean ± SE, *n* = 3.

The release of fenretinide from solubilizer-free Eudragit® RL PO/RS PO films was very slow (13-15 % release after 8 h). Co-encapsulation of single (17-22 and 50-58 % release, respectively, after 1 and 8 h from 20 wt% Tween® 20 and 80 and sodium deoxycholate loaded films) or mixed (24 and 75% release respectively after 1 and 8 h from 20 wt% Tween® 80 + 40 wt% sodium deoxycholate loaded film) solubilizers in fenretinide/Eudragit® RL PO films led to significant improvement in drug-release over a period of 8 h.

### EXAMPLE 2

### Materials

Sodium deoxycholate (Sigma-Aldrich, Co., St. Louis, MO), Tween® 80 (Sigma-Aldrich, Co., St. Louis, MO), Eudragit® RL-PO (Rohm GmbH, Pharma Polymers, Darmstadt, Germany), propylene glycol (MP Biomedicals, LLC, Solon, OH). Fenretinide (MK-4016) was provided by Merck Corp.

Porcine buccal tissue was obtained from local slaughterhouse and used within 2 hours of slaughter. Tissue was kept on ice during transit. The epithelium was separated from the underlying connective tissue with surgical technique.

### Methods for Example 2

### Preparation of oral patch

Various types of mucoadhesive layers were prepared by a casting method. Briefly, about 50 mL polymer solutions (1.5 %w/v) of various mucoadhesive polymers were each prepared in water by stirring overnight and poured into glass Petri dishes. The water was evaporated by incubating the Petri dishes at 50°C for 24 h. The films were then removed and stored in a desiccator until further use.

The drug layer was also prepared by a casting method using Eudragit® RL-PO polymer. Briefly, 12% (w/w) Tween® 80, 33% (w/w) sodium deoxylcholate, 5% (w/w) fenretinide, 50% (w/w) Eudragit® RL-PO and 5% (w/w) or 10% (w/w) permeation enhancers were dissolved in 10 ml acetone: ethanol (50:50). All the weight to weight ratios were based on the total amount of polymer, excipients and drug.

The solution was casted onto Teflon coated Petri dishes. The Petri dishes were then incubated at 37 °C for 24 h. The films were removed and stored in a desiccator at -20°C until further use.

### Determination of fenretinide solubility in bovine serum

Excessive amount of fenretinide was added into fetal bovine serum in polypropylene tubes. The sample was placed on a mechanical circulator and incubated at 37C avoiding light. At every 24 hours (until fenretinide reached saturation in serum), the sample was centrifuged at 8000 rpm for 10 minutes. 0.2 ml sample was drawn from the supernatant. This volume was immediately replaced using blank serum. The tubes were shook to mix the supernatant and sediment, and then re-placed on the mechanical circulator and incubated at 37C. The fenretinide concentration in the supernatant was measured by a HPLC assay after extraction using acetonitrile.

### In vitro permeation

*In vitro* permeation studies were investigated using a side-by-side Franz diffusion cell apparatus. The orifice diameter in both donor and receptor compartments was 1 cm (0.785 cm²). Porcine buccal membrane was mounted between donor and receiver compartments of the diffusion cell. Fenretinide patches were placed on surface side of buccal membrane, in such a way that the backing layer faced the donor chamber and the adhesive film facing the membrane. The receptor compartment held phosphate buffered saline (PBS, pH = 7.4) containing 0.084% Tween 80 (v/v), which was degassed prior to use by vacuum filtration through a HPLC filter. The donor compartment held saliva buffer. Compartment temperature was kept constant at 37°C by recirculating water from a thermostatically controlled bath. Continuous stirring was provided by stirring bar, rotating at 600 rpm. 1 ml samples were collected from the receptor compartment at defined time intervals (1, 2, 3, 4, 5, 6, 7, 8 and 12 h). This volume was immediately replaced using blank, pre-warmed PBS buffer. The samples were then analyzed by HPLC.

### Fenretinide HPLC assay

HPLC assays were performed on a Waters 2695 alliance system (Milford, MA, USA) consisting of a 2996 Photodiode array detector and a personal computer with Empower 2 Software. A symmetry C18 column (4 µm, 150 mm × 4.6 mm) was used. Isocratic elution with acetonitrile: 0.1 % (v/v) phosphoric acid (67:33 v/v) was employed at a flow rate of 1.0 ml/min and detection wavelength was set at 365 nm. Volume of injection was 50 µl. All samples were analyzed at room temperature. Standard curve of fenretinide was established in acetonitrile: ethanol (50:50) and concentration of unknown samples was calculated from the standard curve.

### Results for Example 2

### Fenretinide solubility in bovine serum

The solubility of fenretinide in serum after 6 days incubation was 20.945 ± 1.022 µg/ml. As the intrinsic aqueous solubility of fenretinide is extremely low (0.0098 µg/ml) (practically insoluble in water), the fenretinide concentrations achieved in serum might be fenretinide bound to serum proteins, such as albumin, lipoproteins and serum retinol-binding protein (RBP). RBP, a glycoprotein, is a well-characterized protein which transports retinol in plasma. It consists of a single polypeptide chain of 21 kDa which binds one molecule of retinol. It forms a complex with all-trans retinol (ATRol) in the liver and is involved in the transport of ATRol in the blood. Fenretinide also interacts with RBP to form a tight complex, but the affinity is lower than that of retinol.

The inventors herein have shown that the addition of Tween® 80 can improve the solubility of fenretinide. Therefore, to mimic the physiological conditions inside the body, *in vitro* permeation studies were performed in the presence of 0.084% Tween 80 (v/v) in the receiver containing PBS buffer (pH 7.4) as the solubility of fenretinide in serum is equal to that in 0.084% Tween 80 (v/v) solution.

### Permeation of fenretinide through porcine buccal tissue

Permeation profiles of fenretinide, i.e., cumulative percentages and amounts of fenretinide permeated through porcine buccal mucosa plotted against time, are shown in **Figure 8** and **Figure 9****,** respectively. The **Figures 8-9** show that the co-solvent system described herein enhances oral mucosal permeation of fenretinide. In particular, there is an enhancement of oral mucosal permeation of fenretinide by co-incorporation of propylene glycol (co-solvent) in the hydrogel-based fenretinide controlled release system.

The steady state flux (*Jₛ*), cumulative amounts and percentages of fenretinide in the receiver and tissue, and enhancement factors from PG-incorporated patches are shown in **Table 3.**

| **Table 3** Evaluation of oral mucosal permeation enhancement characteristics of propylene glycol for fenretinide | | | | | | | |
|---|---|---|---|---|---|---|---|
| Formulation | J₅ (µg cm⁻² h⁻¹)*^{a}* | Cumulative amount of fenretinide in the receptor Q₁₂ₕ(µg) | Cumulative percentage of fenretinide in the receptor fluid Q₁₂ₕ(%) | Fenretinide amount in the tissue (µg) | Fenretinide percentage in the tissue (%) | EF for fenretinide in the receptor^{b} | EF for fenretinide in the tissue^{c} |
| Control | 10.03 ± 1.03 | 66.96 ± 3.72 | 2.16 ± 0.12 | 234.05 ± 45.88 | 7.55 ± 1.48 | 1 | 1 |
| Propylene glycol 5% | 16.19 ± 2.21 | 86.49 ± 5.27 | 2.79 ± 0.17 | 504.06 ± 33.79 | 16.26 ± 1.09 | 1.3 | 2.2 |
| Propylene glycol 10% | 22.77 ± 2.90 | 1.12.22 ± 3.10 | 3.62 ± 0.10 | 616.28 ± 40.92 | 19.88 ± 1.32 | 1.7 | 2.6 |
| Propylene glycol 10% + Menthol 5% | 39.75 ± 2.11 | 131.75 ± 4.03 | 4.25 ± 0.13 | 908.92 ± 84.32 | 29.32 ± 2.72 | 2.0 | 3.9 |
| *^{a} Jₛ* steady sate fluxes obtained from the slope of the graph of cumulative amount permeated versus time. (the linear portion of the concentration-time profiles). | | | | | | | |
| *^{b}*Enhancement factor (EF) = cumulative percentage of fenretinide in the receiver from patch with enhancer / cumulative percentage of fenretinide in the receiver from control patch. | | | | | | | |
| *^{c}*Enhancement factor (EF) = fenretinide percentage in the tissue from patch with enhancer / fenretinide percentage in the tissue from control patch. | | | | | | | |

The results in **Table 3** demonstrate that patches containing both 5% and 10% PG increased buccal mucosa permeation and retention of fenretinide. This enhancement was in a greater extent when more PG (10%) was incorporated into the patch. Propylene glycol enters the buccal tissue, competes for the solvation sites of the polar head groups of the lipid bilayers and increases the solubility of this site for the permeant. As a consequence, the partitioning of the drug from the patch into the buccal tissue increases.

When 5% menthol was incorporated into the patch which contained 10% PG, much more drug (3.9 times in comparison with patch without enhancer) was recovered from the buccal tissue. As terpenes can enhance both hydrophilic drugs including propranolol and lipophilic drugs such as testosterone. This permeation enhancement results from the synergistic effect of propylene glycol and menthol. The menthol improves the permeation of drugs by increasing the drug diffusivity in the membrane by modifying the intercellular packing, disrupting highly ordered structure of lipids.

Propylene glycol has an effect on the enhancement of buccal mucosa permeation and retention of fenretinide. The combination of other enhancers with propylene glycol provides a synergistic effect and significantly increases the activity of enhancers. As such, there may also be provided herein methods and buccal patches containing PG and other permeation enhancer (e.g., L-menthol, oleic acid).

### EXAMPLE 3

### Chemicals, Tissue, and Animals.

Fenretinide was received as a gift sample from Merck & Co., Inc. (Whitehouse Station, NJ). Sodium deoxycholate, Tween® 80 and L-menthol were purchased from Sigma-Aldrich, Co. (St. Louis, MO). Noveon® AA-1 polycarbophil (PC), hydroxypropyl methylcellulose (HPMC) 4KM and Eudragit® RL-PO were all gifts from Lubrizol Corp. (Wickliffe, OH), Colorcon®, Inc., (West point, PA), and Evonik Degussa Corp. (Piscataway, NJ), respectively. Propylene glycol was purchased from MP Biomedicals, LLC (Solon, OH). Teflon® overlay was purchased from Scientific Commodities, Inc., (Lake Havasu City, AZ). Tegaderm™ roll was purchased from 3M Health Care (St. Paul, MN). Porcine buccal tissue was obtained from slaughter house (Dunbar Meat Packing Company, Milan, MI, USA). Rabbits were purchased from Harlan Laboratories (Indianapolis, IN, USA).

### Preparation of Oral Mucoadhesive Patches for Enhanced Buccal Permeation of Fenretinide.

Fenretinide/Eudragit® RL-PO/solubilizers patches with and without permeation enhancers (PG and menthol) were prepared by a solvent casting and assembly techniques as described herein. Three steps were involved in the preparation of fenretinide patch: formation of adhesive (hydroxypropyl methylcellulose and polycarbophil at a weight ratio of 3:1) and drug release (5 wt% fenretinide/Eudragit® RL-PO/40 wt% sodium deoxycholate/20wt% Tween® 80) layers, and assembly of adhesive and drug release layers onto backing layer (Tegaderm™ film) (see Figures 1A-1C. The drug release (fenretinide) layer included permeation enhancer(s) in addition to the formulation given above. Eudragit® RL-PO/5 wt% fenretinide/40 wt% sodium deoxycholate/20wt% Tween® 80 layer loaded with PG alone (5 and 10 wt%) or menthol alone (5 and 10 wt%) or in combination (1 wt% PG + 5wt% menthol, 2.5 wt% PG + 5wt% menthol, and 10 wt% PG + 5wt% menthol) were prepared

### Fenretinide HPLC Assay.

HPLC assays were performed on a Waters 2695 alliance system (Milford, MA, USA) consisting of a 2996 Photodiode array detector and a personal computer with Empower 2 Software. A symmetry C18 column (4 µm, 150 mm × 4.6 mm) was used. Isocratic elution with acetonitrile: 0.1 % (v/v) phosphoric acid (67:33 v/v) was employed at a flow rate of 1.0 mL/min and detection wavelength was set at 365 nm. Standard curve of fenretinide was established in acetonitrile: ethanol (50:50) and concentration of unknown samples was calculated from the standard curve.

### Determination of Fenretinide Solubility in Bovine Serum.

A known quantity (0.9, 2.26, 3.97, 8.03, and 20.5 mg) of fenretinide was added to polypropylene tubes containing 15 mL fetal bovine serum. The samples were incubated at 37°C under constant rotation using a rigged rotator and protection from light. At every 24 h till 7 days, the samples were centrifuged at 8000 rpm for 10 minutes and 200 µl of supernatant was withdrawn. Withdrawn serum sample was replaced with fresh serum sample, mixed properly, and incubated again under similar conditions. To the withdrawn sample (200 µl), 2 mL of acetonitrile was added, agitated overnight on a mechanical shaker with protection from light, passed through 0.45 µm PVDF filter units, and analyzed by HPLC.

### Determination of Fenretinide Loading.

Fenretinide/Eudragit® films were digested in acetonitrile: ethanol (50:50), passed through 0.45 µm PVDF filter units, and analyzed by HPLC after suitable dilution. The fenretinide loading was calculated as the percentage of the amount of fenretinide versus the total weight of the film mixture (i.e., fenretinide, Eudragit®, and other excipients).

### Evaluation of In vitro Release of Fenretinide from Oral Mucoadhesive Patches.

Simulated saliva comprised of 14.4, 16.1, 1.3, 0.55, and 2 mM sodium chloride, potassium chloride, calcium chloride dihydrate, magnesium chloride hexahydrate, and dibasic potassium phosphate and the pH was adjusted to 6.8. *In vitro* release studies were conducted in simulated saliva containing 5% (w/v) sodium deoxycholate under perfect sink conditions. Mucoadhesive patches were placed in 50 mL tubes (separate tubes for each sampling interval) and 40 mL release medium was added to each tube. The tubes were placed in an incubator maintained at 37°C and shaken at 100 RPM. At predetermined time intervals (0.5, 3, and 6 h), tubes were taken out and the patches were immediately freeze-dried. The amount of fenretinide remaining in the patch was determined as per the method described in loading assay. The cumulative amount of fenretinide released was calculated by subtracting the fraction remaining in the patches from the initial drug content.

### Ex vivo Permeation of Fenretinide across Porcine Buccal Mucosa.

*Ex vivo* permeation of fenretinide across porcine buccal mucosa was conducted using side-by-side flow-through diffusion cells (donor and receiver chamber volume = 3 mL). The diffusional interface was a spherical shape with a diameter of 1 cm. Porcine buccal tissue was obtained from a local slaughterhouse and used within 2 hours of slaughter. The tissue was stored in Krebs buffer at 4°C upon removal. The epithelium was separated from the underlying connective tissue with a scalpel and mounted between the donor and the receiver chambers. Fenretinide patch was then attached to the buccal mucosa (adhesive layer facing mucosa and backing layer exposed to buffer) in donor chamber. Donor and receiver chambers were filled with 3 mL degassed phosphate buffered saline (PBS, pH = 7.4) containing 0.084% Tween® 80 (v/v) and simulated saliva (pH 6.8), respectively. Both the chambers were maintained at 37°C by circulating the water from a thermostatically controlled water bath. The receiver chamber medium was stirred at 600 rpm. After specified duration (1, 2, 3, 4, 5, 6, 7, 8 and 12 h), 1 mL sample was withdrawn from the receiver chamber and immediately replaced with fresh medium. Fenretinide was quantified by HPLC. At the end of permeation study, phenol red at a concentration of 300 µg/ml was added to the donor chamber to check the integrity of buccal mucosa. Phenol red acts as a marker compound, which does not permeate through an intact porcine buccal membrane. Upon the completion *of ex vivo* permeation study, porcine buccal tissue was removed and fenretinide level in the tissue was determined as described below.

### Determination of Fenretinide Levels in Buccal Tissue.

Treated porcine buccal tissue was cut into small pieces and placed in 4-mL polypropylene tubes. One milliliter of water was added to the tubes and homogenized for 1 minute. Then, 2 mL of acetonitrile was added to the tubes and vortexed for 1 hour. After 1 h, tubes were centrifuged at 2600 g at 25°C for 20 min and the supernatant was analyzed by HPLC to determine fenretinide content.

### Haematoxvlin and Eosin Staining.

A portion of each tissue was fixed in buffered 10% formalin and embedded in paraffin wax. Then, 5 µm sections were placed on microscope slides, deparaffinized using xylene, and rehydrated using ethanol solutions in a gradient of 80% up to 100% and distilled water. The tissue slices were placed in 0.7% w/w haematoxylin solution, rinsed twice in acid ethanol (0.1 N HCl in 95% ethanol) to remove the excess stain. Subsequently, the tissue slices were placed in 0.1% w/w eosin solution and dehydrated using solutions of ethanol in a gradient of 80% up to 100% and then xylene.

### Light Microscopy Analysis.

Light microscopy was performed using Olympus BX51 microscope (Olympus, Tokyo, Japan) at 40 × magnification. Images of the sections were captured using a fitted camera (Olympus DP70 digital camera, Tokyo, Japan), and software (Olympus DP controller, Tokyo, Japan).

### Evaluation of In vivo Fenretinide Release and Permeation.

Animal studies were approved by the Ohio State University Institutional Animal Care and Use Committee and adhered to National Institute of Health guidelines. Female New Zealand white rabbits (12 weeks old and weight ranging 2.7-3.1 kg) were anesthetized with isoflurane (5% v/v in oxygen) via inhalation for patch placement and removal. Six fenretinide oral mucoadhesive patches/time point were placed on the buccal mucosa of subject rabbit's oral cavity (drug + adhesive layers facing the mucosa). Slight pressure was applied to the backing layer of the patch for 1 minute to establish mucoadhesion with the rabbit buccal mucosa. After different attachment times (0.5, 3 and 6 h), the patches were carefully removed and remaining fenretinide in patches was determined by HPLC. The cumulative amount of fenretinide released was determined by subtracting the fraction remaining in the patches from the initial drug content. To determine the drug level in the tissue, extraction and quantitation of fenretinide was performed as described in the determination of fenretinide level in buccal tissue section.

### Statistical Analysis.

The results are expressed as mean ± SE *(n* = 3/4 *(in vitro)* or 5 *(ex vivo)* or 6 *(in vivo)).* An unpaired Student's t-test and one-way ANOVA were used to compare the means of *in vitro* and *in vivo* drug release, *ex vivo* porcine buccal mucosal permeation and tissue levels of fenretinide, *in vivo* tissue levels of fenretinide and assess statistical significance. Results were considered statistically significant if *p*<0.001.

### Discussion of Example 3

### Mucoadhesive Fenretinide Patches with Enhanced Drug Permeability.

As described herein, the mucoadhesive patch formulation of fenretinide was tested for site-specific chemoprevention of oral cancer. Solubilizer-free patches exhibited poor *in vitro* and *in vivo* drug release behavior. Co-incorporation of either single or mixed solubilizers (e.g., Tween® 20 and 80, sodium deoxycholate) in fenretinide/Eudragit® patches led to significantly improved continuous *in vitro* and *in vivo* fenretinide release. In the past, the use of fenretinide in chemoprevention of oral cancer has been hindered by several key limitations, e.g., poor solubility, biological membrane permeability and bioavailability, and rapid elimination of drug from the body. Undesired effects are rendered mainly by its extremely high hydrophobicity (log *P* = 8.03) and low water solubility (below detection limit).

Fenretinide-loaded Eudragit® RL PO layers with and without permeation enhancers were prepared by a solvent casting method with drug loading efficiency of 90-95 %, as seen in Table 4.

**Table 4. Evaluation of Microencapsulation of Fenretinide in Permeation Enhancer-Free and Permeation Enhancers-Loaded Eudragit® RL-PO Films.**

| Patch formulation | Fenretinide loading (wt%) | | Loading efficiency (%) |
|---|---|---|---|
| | Theoretical^{a} | Actual^{*} | |
| Permeation enhancer-free | 5.26 | 4.76 ± 0.06 | 90.44 ± 1.14 |
| 5 wt% PG | 5.00 | 4.59 ± 0.07 | 91.81 ± 1.40 |
| 10 wt% PG | 4.76 | 4.52 ± 0.12 | 94.94 ± 2.52 |
| 5 wt% Menthol | 5.00 | 4.62 ± 0.06 | 92.40 ± 1.20 |
| 10 wt% Menthol | 4.76 | 4.51 ± 0.11 | 94.71 ± 2.31 |
| 1 wt% PG + 5 wt% Menthol 2.5 | 4.95 | 4.48 ± 0.13 | 90.50 ± 2.63 |
| wt% PG + 5 wt% Menthol 10 | 4.88 | 4.53 ± 0.11 | 92.87 ± 2.26 |
| wt% PG + 5 wt% Menthol | 4.54 | 4.14 ± 0.05 | 91.10 ± 1.10 |

| | | | |
|---|---|---|---|
| * Mean ± SE, *n* = 3; ^{a}Based on polymer + excipients weight | | | |

The thickness of fenretinide and adhesive layers, and the Tegaderm™ adhesive film were measured to be ∼ 0.28, 0.28, and 0.05 mm, respectively. After assembling drug and adhesive layers onto backing layer, the total thickness of the patch was measured to be ∼ 0.33 mm.

### Determination of Optimal Quantity of Surfactant to Maintain Ex vivo Sink Condition for Fenretinide: Solubility of Fenretinide in Bovine Serum.

Sink condition is one of the key features that govern *in vitro* release or *ex vivo* biological membrane permeability of hydrophobic drugs. The process *of ex vivo* drug transport from the patch (donor compartment) to receiver medium (receiver compartment) involves release of drug from the patch to the buccal surface, permeation of drug into the buccal tissue, and release (after dissolution if necessary) of drug from the tissue into the receiver chamber medium.

The patch described herein is useful for extremely hydrophobic fenretinide, and comprises an effective amount on one or more solubilizers to facilitate continuous *in vitro* and *in vivo* fenretinide release and tissue permeation enhancers to improve fenretinide permeability across buccal mucosa. To maintain a desired sink condition in the release/receiver chamber medium, appropriate quantity of suitable solubilizing agent can be incorporated. In this Example, the optimal quantity of nonionic surfactant in the release media was selected by matching the drug solubility to that in bovine serum.

The solubility of fenretinide in bovine serum at different fenretinide concentrations (0.9, 2.26, 3.97, 8.03 and 20.5 mg) and incubation times (1-7 days) is shown in **Figures 10A-1010B**. The solubility of fenretinide in bovine serum was found to be 21 ± 1 µg/mL **(****Figure 10A**). Bovine serum comprises numerous proteins namely albumin, lipoproteins and serum retinol-binding protein (RBP). Enhanced solubility of fenretinide in bovine serum can be attributed to protein-drug binding or complexation.

The time taken by fenretinide to reach equilibrium with bovine serum was affected by the amount of fenretinide added in bovine serum. For example, when the amount of fenretinide was increased from 0.9 to 8.03 mg, the time required to achieve equilibrium was reduced from 7 to 4 days **(****Figure 10B****).** Further increases in fenretinide quantity did not reduce the time required for equilibration, thereby suggesting the necessity of minimum ∼8 mg of fenretinide and 4 days incubation time to reach equilibrium state with 15 mL serum. A concentration of 0.084% Tween® 80 required to reach equivalent solubility of fenretinide (21 µg/mL in bovine serum) in test medium (receiver chamber medium i.e., PBS, pH 7.4) was then determined from the perfect linear relationship of fenretinide solubility in PBS versus Tween® 80 concentration above the surfactant critical micelle concentration. Hence, PBS + 0.084% Tween® 80 was then used to mimic physiological solubilization/sink condition in the *ex vivo* drug permeation studies.

### Enhanced Ex vivo Porcine Buccal Mucosal Permeation of Fenretinide by Co-incorporation of Propylene Glycol and Menthol in Fenretinide/Eudragit® RL-PO Patches.

The effect of co-incorporation of single (5 and 10 wt% PG or menthol) and mixed (1 wt% PG + 5 wt% menthol, 2.5 wt% PG + 5 wt% menthol or 10 wt% PG + 5 wt% menthol) permeation enhancers in fenretinide/Eudragit® RL PO mucoadhesive patches on *ex vivo* porcine buccal mucosal permeation of fenretinide is shown in **Figures 11A-11C****.** *Ex vivo* permeation of fenretinide increased steadily over a period of 8 h and then reached a plateau thereafter. The both the flux *(Js)* at steady state and the enhancement factor (EF = *Js* with enhancer/*Js* without enhancer) were calculated.

The fraction of drug permeated across buccal mucosa and deposited in the buccal tissue, and values of *Js* and EF are given in Table 5.

**Table 5. Evaluation of Potential of Co-incorporation of Permeation Enhancers (Propylene Glycol (PG), Menthol or PG + Menthol) in Fenretinide/Eudragit® RL PO Patches to Enhance Porcine Buccal Mucosal Permeation of Fenretinide Ex Vivo.**

| Patch formulation | Flux (*Jₛ*) (µg cm⁻² h⁻¹)*^{a}* | Fenretinide in the receptor medium (µg/mL) | Fenretinide in EF*_{b}* the tissue (µg/g) |
|---|---|---|---|
| Permeation enhancer-free | 10.0 ± 0.5 | 22.3 ± 0.5 | 43.8 ± 6.11.0 |
| 5 wt% PG | 16.2 ± 0.9 | 28.8 ± 0.8 | 158.5 ± 4.7 1.6 |
| 10wt% PG | 22.8 ± 1.3 | 37.4 ± 0.5 | 170.7 ± 5.3 2.3 |
| 5 wt% Menthol | 12.4 ± 0.6 | 22.8 ± 0.5 | 61.7 ± 5.11.2 |
| 10 wt% Menthol | 12.7 ± 0.7 | 23.5 ± 0.7 | 65.3 ± 4.7 1.3 |
| 1 wt% PG + 5 wt% Menthol | 17.9 ± 0.6 | 35.8 ± 1.0 | 172.1 ± 7.6 1.8 |
| 2.5 wt% PG + 5 wt% Menthol | 20.2 ± 0.8 | 39.4 ± 0.8 | 175.6 ± 7.0 2.0 |
| 10 wt% PG + 5 wt% Menthol | 39. 8 ± 0.9 | 43.9 ± 0.6 | 241.1 ± 9.8 4.0 |

| | | | |
|---|---|---|---|
| *ₐJₛ* Steady state flux was calculated from linear regression of cumulative amount permeated vs. time; *_{b}*Enhancement factor (EF) = *Jₛ* in the presence of permeation enhancer/*Jₛ* in the absence of permeation enhancer; Values represent mean ± SE, *n* = 5. | | | |

Co-incorporation of single (**Figure 11A** and **Figure 11B**) or mixed **(****Figure 11C****)** permeation enhancers in the patch led to significant enhancement (p < 0.001) in the rate and extent of fenretinide permeation across porcine buccal mucosa (see Table 5).

For example, the flux for permeation enhancer-free patch was found to be ∼ 10 µg cm⁻² h⁻². After co-incorporation of 10 wt% PG or 10 wt% PG + 5 wt% menthol, the flux was increased to ∼ 23 (EF = 2.3) and 40 (EF = 4) µg cm⁻² h⁻², respectively. In contrast, a slight increase in the flux was observed with menthol patch formulations (Js = ∼ 13 µg cm⁻² h⁻²). The levels of drug in tissue were in agreement with the values of flux (see Table 5). Fenretinide content in buccal tissue after 12 h of *ex vivo* permeation with permeation enhancer-free patch was found to be ∼ 44 µg/g. Co-incorporation of PG or PG + menthol led significantly high amount of fenretinide recovery from the buccal tissue (∼ 171 and 241 µg fenretinide/g tissue with 10 wt% PG and 10 wt% PG + 5 wt% menthol formulation, respectively), thereby indicating increased tissue localization/permeation of fenretinide in the presence of PG or PG + menthol. A moderate enhancement effect was exhibited by menthol alone.

Propylene glycol exerts its permeation enhancement effect by competing for the solvation sites of the polar head groups of the lipid bilayers and occupying the hydrogen bonding sites, thereby increasing the solubility of this site for the permeant. PG may increase the lipid fluidity which in turn facilitates enhanced drug permeation. Enhanced permeation of fenretinide in the presence of PG can be attributed to one or both of these mechanisms. Menthol, on the other hand, has the ability to modify the drug diffusivity and/or partitioning by disrupting the conformational order of the intercellular lipids in bilayers. Menthol alone it did not provide significant permeation enhancement of fenretinide (p > 0.001). This result can be attributed at least in part to non-homogeneous distribution of menthol in fenretinide/Eudragit® RL PO matrix (see Figure ID) due to crystallization and aggregation of menthol during solvent evaporation.

When PG was combined with menthol, this issue was overcome (see Figure ID) and pronounced fenretinide permeation enhancement was observed relative to menthol alone (see **Figure 11C** and Table 5). Desirable fenretinide permeation observed with mixed permeation enhancers (PG + menthol) can be attributed to synergistic effect between menthol and PG.

### Morphological and Histological Characteristics.

Photomicrographs of the sections of buccal mucosa are shown in Figures 12A-12H. The porcine buccal mucosa, similar to human buccal mucosa, consists of an outermost layer of keratinized stratified squamous epithelium, below which lies a basement membrane, a lamina propria followed by the submucosa containing the buccinator muscle as the innermost layer. Regardless of patch application, all sections showed an appropriately maturing stratified squamous epithelium. Scattered mitotic figures were restricted to the basilar layers, and the outermost granular and corneal layers showed appropriate terminal differentiation as reflected by surface parakeratin production. No evidence of changes consistent with extensive epithelial perturbations attributable to a contact mucositis, e.g., hydropic degeneration of the basal cell layer or acantholysis were noted.

Basal epithelial cells are tightly bound together in the control (no patch attachment) sample (see **Figure 12A**). Noticeable morphological changes (e.g., prickle cells) in the underlying layers and significant loss of superficial cell layers were not apparent after attachment of 5 wt% (see **Figure 12B**) and 10 wt% (see **Figure 12C**) wt% PG loaded patches. An increase in intercellular edema and swelling of buccal epithelium are visible, however, in **Figure 12B** and **Figure 12C** when the loading of PG is above 5 wt%.

The photomicrographs of buccal epithelium after treatment with 5 and 10 wt% menthol loaded patches are respectively shown in **Figure 12D** and **Figure 12E****.** It is visible that the epithelium layers were intact in both the samples. In addition, there was no sign of cellular swelling and significant histological and ultrastructural changes.

Similar results were observed in samples treated with 1 wt% PG + 5 wt% menthol (see **Figure 12F**) and 2.5 wt% PG + 5 wt% menthol (see **Figure 12G**) loaded patches. In contrast, tissue exposed to the 10 wt% PG + 5% menthol loaded patch showed a moderate increases in intracellular space and intercellular edema (see **Figure 12H**). Since menthol did not cause any epithelial cell alteration, it is likely that higher (10 wt %) loading of PG in the patch resulted in increased intracellular space and intercellular edema.

The histological changes (e.g., increases in intracellular space and intercellular edema) observed in the tissues treated with 5 and 10 wt% PG loaded patches (see **Figure 12B, Figure 12C** and **Figure 12H**) are indicative of diffusion of PG into subject keratinocytes as well as the intercellular spaces. Upon permeation and accumulation in cells, it is likely that PG interacted with intercellular or membrane lipids, thereby increasing the permeability of fenretinide through epithelium. Since 2.5 wt% PG + 5 wt% menthol loaded patches exhibited optimal drug permeation enhancement with no morphological and histological changes, this formulation was selected and used to further evaluate *in vivo* release, permeation and tissue deposition kinetics of fenretinide, as described below.

### In vitro and In vivo Release Characteristics ofPermeation Enhancers-Loaded Fenretinide/Eudragit® RL-PO Patches.

*In vitro* and *in vivo* fenretinide release from permeation enhancer-free and 2.5 wt% PG + 5 wt% menthol-loaded fenretinide/Eudragit® RL-PO patches is shown in **Figure 13****.** Both the patch formulations provided continuous *in vitro* and *in vivo* fenretinide release from Eudragit® polymeric matrices, and the addition of PG and menthol did not significantly affect the release kinetics, indicating further fenretinide solubilization and/or changes to the patch swelling behavior. In this case, the patch release characteristics were largely determined by sodium deoxycholate and Tween® 80, which served as the effective solubilization role in the patch formulation

There was a significant difference *(p* < 0.001) between *in vitro* and *in vivo* fenretinide release characteristics of permeation enhancer-free and 2.5 wt% PG + 5 wt% menthol-loaded fenretinide/Eudragit® RL-PO patches (see **Figure 13**), although the continuous release trend was the same. This difference can be linked to dissimilarity in test conditions (e.g., *in vitro* drug release in simulated saliva vs. *in vivo* drug release followed by permeation across buccal mucosal membrane).

### Enhanced In vivo Rabbit Buccal Mucosal Permeation and Deposition of Fenretinide by Co-incorporation of Propylene Glycol and Menthol in Fenretinide/Eudragit® RL-PO Patches.

The effect of co-incorporation of permeation enhancers (PG + menthol) in fenretinide/Eudragit® RL-PO patches on *in vivo* buccal mucosal permeation and deposition of fenretinide is shown in **Figure 14****.** The level of fenretinide in rabbit buccal tissue increased steadily as function of attachment time of both the patch (permeation enhancer-free and permeation enhancers-loaded patches) formulations (see **Figure 14****),** thereby indicating excellent efficacy of these patch formulations to provide continuous *in vivo* fenretinide permeation across the rabbit buccal mucosa. The extent of fenretinide permeation and tissue deposition provided by 2.5 wt% PG + 5 wt% menthol-loaded patches was significantly higher (43.0 ± 7.7 µg fenretinide/g tissue after 6 h of attachment) than that of permeation enhancer-free patch (17.3 ± 0.3 µg fenretinide/g tissue after 6 h of attachment) (see **Figure 14**). These results show excellent effectiveness of co-incorporation of PG and menthol to obtain improved oral mucosal permeation and tissue levels of fenretinide. Different permeation and tissue deposition kinetics of fenretinide obtained with *ex vivo* and *in vivo* studies can be attributed to dissimilarity in key test conditions (e.g., porcine vs. rabbit buccal mucosas, *ex vivo* vs. *in vivo* sink conditions).

These data demonstrate the therapeutic advantage imparted by mucoadhesive patch local delivery of fenretinide, i.e., obtaining pharmacologically active levels in the target tissue. *In vitro* fenretinide concentrations between 1 and 10 µM are useful for inducing desirable chemopreventive effects, e.g., cellular terminal differentiation (<3 µM) and apoptosis (>5 µM). As shown herein, the levels of fenretinide delivered to rabbit buccal mucosa from permeation enhancers loaded patch ranged from 7.75 µg/g (0.5 hour; 19.8 µM) to 42.36 µg/g (6 hours; 108.2 µM). Therefore, short duration patch application (i.e., less than 30 minutes) may be especially useful in order to provide therapeutically relevant concentrations in the targeted oral epithelium. In addition, due to the decreased treatment time, such applications can facilitate patient compliance.

The intraoral site-specific fenretinide delivery us thus enhanced by the mucoadhesive patches that provide enhanced buccal mucosal permeation and tissue levels of fenretinide. Suitable permeation enhancers (PG and menthol) were co-incorporated in fenretinide/Eudragit® RL-PO patches. Mucoadhesive patches containing a desired drug delivery (fenretinide + solubilizers + permeation enhancers), adhesive, and backing layers were prepared by solvent casting and assembling techniques. Co-incorporation of PG or PG + menthol in patches led to significant *ex vivo* and *in vivo* buccal mucosal permeation and tissue deposition of fenretinide, a extremely hydrophobic and poorly tissue permeable chemopreventive agent. A mucoadhesive patch co-incorporated with 2.5 wt% PG + 5 wt% menthol was found to be have desired oral mucosal permeation enhancement without significantly affecting the observed histology of the oral mucosa.

### Formulations for use in Methods of Treatment

The formulations disclosed herein are generally used by applying the formulations topically to mucosal surfaces of the oral cavity.

According to one aspect of the present invention, the formulation of the present invention is used in a method comprising: i) providing a transmucosal system comprising said formulation; ii) applying the transmucosal system to a mucosal membrane of a subject; and, iii) keeping the transmucosal system in contact with the mucosal membrane for a therapeutically effective period of time; and, iv) optionally removing the transmucosal system when a desired therapeutic effect has been achieved.

In certain embodiments, the patch contains a permeation enhancer agent that is not present an adhesive layer, but is only present in the formulation.

According to another aspect of the present invention, the formulation of the present invention is used in a method of treatment and prophylaxis of a disease or disorder, comprising: administering said formulation to a subject in need of such treatment.

In certain embodiments, the formulation can be present as a mouth product such as a toothpaste, a mouthwash or mouth rinse, a gel or paste, a spray, a chewing gum, and/or a lozenge.

### EXAMPLE 3

### Therapeutic Uses

The formulations described herein have useful clinical applications for preventing development (primary chemoprevention) or inhibiting recurrence (secondary chemoprevention) of oral cancer.

Another therapeutic use includes treatment for includes inhibiting growth of a tumor such as head and neck squamous carcinoma cells (HNSCC).

Another therapeutic treatment includes decreasing the size of a tumor, comprising tumor cells, wherein the tumor cells are head and neck squamous cell carcinoma cells.

Another therapeutic treatment includes preventing head and neck squamous cell carcinoma (HNSCC).

Another clinical application is the use of the formulations on actinically induced precancerous lesions of the lower lip, known as actinic cheilitis. While not as clinically aggressive as intraoral dysplastic lesions, lip lesions need to be surgically managed, and can progress into oral squamous cell carcinoma.

Still other clinical applications can include all variants of oral squamous cell carcinoma (including these actinically-induced lip lesions) as treatment sites.

Other clinical applications include the treatment, amelioration or reversal of oral epithelial dysplasias, such as Fanconi anemia.

### Kits

Also provided herein are kits comprising the formulations described herein and instructions for use in a method for administering to a subject.

The kit may include instructions for use in the treatment of a cancerous or precancerous condition. The kit may include instructions for administering the composition to a mammal with a head or neck basal cell precancerous or cancerous condition. It is to be understood that the formulations described herein can be packaged in kit form. A kit may include delivery systems in suitable packaging.

Each formulation is supplied in a pharmaceutically acceptable carrier that is suitable for inventory storage. A kit may optionally provide additional components that are useful in the methods and formulation procedures disclosed herein, such as buffers, reacting surfaces, or means of purifying delivery particles.

In addition, the kits optionally include labeling and/or instructional or interpretive materials providing directions (i.e., protocols) for the practice of the methods disclosed herein, such as preparation, formulation and/or use of delivery particles. While the instructional materials typically comprise written or printed materials they are not limited to these formats. Any medium capable of storing such instructions and communicating them to an end user may be used. Such media include, but are not limited to electronic storage media (e.g., magnetic discs, tapes, cartridges, chips), optical media (e.g., CD ROM), and the like. Such media may include addresses to Internet sites that provide such instructional materials.

Provided herein is a prophylactic kit for reducing the likelihood of disease in a subject comprising: (a) an adhesive pharmaceutical formulation as described herein; and (b) a delivery system, such as a patch or film for delivery of the formulation.

Provided herein is a therapeutic kit for treating disease in a subject comprising: (a) an adhesive pharmaceutical formulation as described herein; and (b) a delivery system, such as a patch or film for delivery of the formulation.

## Claims

1. A formulation, comprising: at least one mucoadhesive material; at least an effective amount of fenretinide or a pharmaceutically acceptable salt thereof; and at least one transmucosal permeation enhancer agent comprising:
propylene glycol (PG) and menthol; and at least one solubilizer agent.

2. The formulation of claim 1, wherein the fenretinide or a pharmaceutically acceptable salt thereof is present at about 0.1 wt% to about 5 wt%.

3. The formulation of claim 1 or 2, wherein the formulation comprises 5 wt% fenretinide or a pharmaceutically acceptable salt thereof.

4. The formulation of claim 1, 2 or 3, wherein the transmucosal permeation enhancer agent comprises from about 1 wt% to about 2.5 wt% PG, and from 1 wt% to about 5 wt% menthol.

5. The formulation of any one of claims 1 to 4, wherein the at least one solubilizer agent comprises one or more of deoxycholic acid, a polyoxyethylene-sorbitan higher fatty acid ester, and combinations thereof.

6. The formulation of claim 5, wherein the polyoxyethylene-sorbitan higher fatty acid ester is selected from polysorbate 20, polysorbate 80, and combinations thereof.

7. The formulation of any one of claims 1 to 4, wherein the at least one solubilizer agent comprises one or more of polysorbate 80 and sodium deoxycholate.

8. The formulation of any one of the preceding claims, wherein the fenretinide or a pharmaceutically acceptable salt thereof and the at least one permeation enhancer agent are adapted to be in contact with at least one common mucosal membrane, optionally wherein the mucosal membrane is the buccal mucosa.

9. The formulation of any one of the preceding claims, present as a mouth product such as a toothpaste, a mouthwash or mouth rinse, a gel or paste, a spray, a chewing gum, and/or a lozenge.

10. The formulation of any one of the preceding claims, wherein the formulation contains a predetermined amount of fenretinide or a pharmaceutically acceptable salt thereof in an amount selected from the group consisting of 10 µg, 15 µg, 25 µg, 50 µg, 100 µg, 500 µg, 1 mg, 2 mg, 3 mg, 4 mg, 5 mg, 6 mg, 7 mg, 8 mg, 9 mg and 10 mg and an adhesive material, the adhesive material providing for adherence to the oral mucosal membrane of a subject.

11. The formulation of any one of the preceding claims, wherein the amount of the fenretinide or a pharmaceutically acceptable salt thereof absorbed via the oral mucosa is selected from the group consisting of at least 35%, at least 40%, at least 45%, at least 50%, at least 55%, at least 60%, at least 65%, at least 70%, at least 75%, at least 80%, at least 85%, at least 90%, at least 95%, at least 98% and at least 99% of the drug in the dosage form.

12. A transmucosal drug delivery system comprising at least one drug-release layer comprised of the formulation of any one of the preceding claims, at least one adhesive layer, and at least one backing layer.

13. A drug dosage form for oral transmucosal administration, comprising:
a formulation comprised of: at least an effective amount of fenretinide or a pharmaceutically acceptable salt thereof, and at least one transmucosal permeation enhancer agent selected from propylene glycol (PG) and menthol; and
an adhesive material, the adhesive material providing for adherence of the drug dosage form to the oral mucosal membrane.

14. The formulation, transmucosal drug delivery system or drug dosage form for oral transmucosal administration of any one of the preceding claims for use in a method of treatment and prophylaxis of a cancerous or precancerous condition, said use comprising: administering said formulation to a subject in need of such treatment.

15. The formulation, transmucosal drug delivery system or drug dosage form for oral transmucosal administration for use according to claim 11, wherein the cancerous or precancerous condition comprises one or more of: oral squamous cell carcinoma, intraoral dysplastic lesions, head and neck squamous carcinoma.

16. The formulation of any one of claims 1 to 11 for use in a method, comprising:
i) providing a transmucosal system comprised of said formulation;
ii) applying the transmucosal system to a mucosal membrane of a subject, optionally the buccal mucosa; and,
iii) keeping the transmucosal system in contact with the mucosal membrane for a therapeutically effective period of time; and,
iv) optionally removing the transmucosal system when a desired therapeutic effect has been achieved.

17. The formulation for use according to claim 16, wherein the transmucosal system includes an adhesive material, optionally wherein the formulation and the adhesive material are present in separate compartments.

18. A method for making a buccal drug delivery system, comprising:
i) preparing a drug-release layer comprised of the formulation, transmucosal drug delivery system or drug dosage form for oral transmucosal administration of any one of claims 1 to 15;
ii) preparing an adhesive layer; and,
iii) assembling the drug layer and the adhesive layers onto a backing layer.

19. A drug dosage form for increasing permeation of fenretinide or a pharmaceutically acceptable salt thereof into a mucosa of a subject in need thereof, the drug dosage form being prepared by:
i) admixing fenretinide or a pharmaceutically acceptable salt thereof with a permeation enhancer agent comprised of one or more of propylene glycol and menthol, and
ii) forming the admixture into a drug-release layer.

20. A method for making the formulation according to any one of claims 1 to 15, comprising:
i) mixing a quantity of at least one solubilizer, at least one mucoadhesive polymer, and at least one permeation enhancer in a solvent to form a solvent mixture;
ii) adding a quantity of fenretinide or a pharmaceutically acceptable salt thereof to the solvent mixture of step i); and, optionally adjusting a volume thereof to 10 mL with the solvent mixture of step i);
iii) forming a layer of the fenretinide or a pharmaceutically acceptable salt thereof mixture of step ii); and,
iv) drying the layer of step iii).

21. A mucoadhesive patch comprising the formulation of any one of claims 1 to 11.

## Patentansprüche

1. Formulierung, umfassend: zumindest ein mucoadhäsives Material; zumindest eine wirksame Menge Fenretinid oder eines pharmazeutisch akzeptablen Salzes davon; und zumindest ein Mittel zur Erhöhung der transmukosalen Permeation, umfassend:
Propylenglykol (PG) und Menthol; und zumindest ein Solubilisierungsmittel.

2. Formulierung nach Anspruch 1, wobei das Fenretinid oder ein pharmazeutisch akzeptables Salz davon mit etwa 0,1 Gew.-% bis etwa 5 Gew.-% vorhanden ist.

3. Formulierung nach Anspruch 1 oder 2, wobei die Formulierung 5 Gew.-% Fenretinid oder ein pharmazeutisch akzeptables Salz davon umfasst.

4. Formulierung nach Anspruch 1, 2 oder 3, wobei das Mittel zur Erhöhung der transmukosalen Permeation etwa 1 Gew.-% bis etwa 2,5 Gew.-% PG und 1 Gew.-% bis etwa 5 Gew.-% Menthol umfasst.

5. Formulierung nach einem der Ansprüche 1 bis 4, wobei das zumindest eine Solubilisierungsmittel eines oder mehrere aus Deoxycholsäure, einem höheren Polyoxyethylensorbitan-Fettsäureester und Kombinationen davon umfasst.

6. Formulierung nach Anspruch 5, wobei der höhere Polyoxyethylensorbitan-Fettsäureester ausgewählt ist aus Polysorbat 20, Polysorbat 80 und Kombinationen davon.

7. Formulierung nach einem der Ansprüche 1 bis 4, wobei das zumindest eine Solubilisierungsmittel eines oder mehrere von Polysorbat 80 und Natriumdesoxycholat umfasst.

8. Formulierung nach einem der vorhergehenden Ansprüche, wobei das Fenretinid oder ein pharmazeutisch akzeptables Salz davon und das zumindest eine Mittel zur Erhöhung der Permeation so angepasst sind, dass sie in Kontakt mit zumindest einer gemeinsamen Schleimhautmembran stehen, wobei die Schleimhautmembran gegebenenfalls die Bukkalschleimhaut ist.

9. Formulierung nach einem der vorhergehenden Ansprüche, die als Mundprodukt wie beispielsweise Zahnpasta, Mundwasser oder Mundspülung, Gel oder Paste, Spray, Kaugummi und/oder Lutschtablette vorliegen.

10. Formulierung nach einem der vorhergehenden Ansprüche, wobei die Formulierung eine vorbestimmte Menge Fenretinid oder eines pharmazeutisch akzeptablen Salzes davon in einer Menge enthält, die aus der Gruppe ausgewählt ist, bestehend aus 10 µg, 15 µg, 25 µg, 50 µg, 100 µg, 500 µg, 1 mg, 2 mg, 3 mg, 4 mg, 5 mg, 6 mg, 7 mg, 8 mg, 9 mg und 10 mg und einem Haftmaterial, wobei das Haftmaterial für die Haftung an der Mundschleimhaut einer Testperson sorgt.

11. Formulierung nach einem der vorhergehenden Ansprüche, wobei die Menge des Fenretinids oder eines pharmazeutisch akzeptablen Salzes davon, die über die Mundschleimhaut absorbiert wird, aus der Gruppe ausgewählt wird, bestehend aus zumindest 35 %, zumindest 40 %, zumindest 45 %, zumindest 50 %, zumindest 55 %, zumindest 60 %, zumindest 65 %, zumindest 70 %, zumindest 75 %, zumindest 80 %, zumindest 85 %, zumindest 90 %, zumindest 95 %, zumindest 98 % und zumindest 99 % des Arzneimittels in der Dosisform.

12. Transmukosales Arzneistoffabgabesystem, umfassend zumindest eine Arzneistofffreisetzungsschicht, die aus der Formulierung eines der vorhergehenden Ansprüche besteht, zumindest eine Haftschicht und zumindest eine Trägerschicht.

13. Arzneimitteldosierungsform zur oralen transmukosalen Verabreichung, umfassend:
eine Formulierung, umfassend: zumindest eine wirksame Menge Fenretinid oder eines pharmazeutisch akzeptablen Salzes davon und zumindest ein Mittel zur Steigerung der transmukosalen Permeation, ausgewählt aus Propylenglykol (PG) und Menthol; und
ein Haftmaterial, wobei das Haftmaterial für die Haftung der Arzneimitteldosierungsform an der Mundschleimhaut sorgt.

14. Formulierung, transmukosales Arzneimittelabgabesystem oder Arzneimitteldosierungsform zur oralen transmukosalen Verabreichung eines der vorhergehenden Ansprüche zur Verwendung in einem Verfahren zur Behandlung und Prophylaxe eines kanzerösen oder präkanzerösen Zustands, wobei die Verwendung umfasst: Verabreichung der Formulierung an eine Testperson, die einer solchen Behandlung bedarf.

15. Formulierung, transmukosales Arzneimittelabgabesystem oder Arzneimitteldosierungsform zur oralen transmukosalen Verabreichung zur Verwendung nach Anspruch 11, wobei der kanzeröse oder präkanzeröse Zustand eines oder mehrere der folgenden umfasst: orales Plattenepithelkarzinom, intraorale dysplastische Läsionen, Plattenepithelkarzinom des Kopfes und Halses.

16. Formulierung nach einem der Ansprüche 1 bis 11 zur Verwendung in einem Verfahren, umfassend:
i) Bereitstellen eines transmukosalen Systems, das aus dieser Formulierung besteht;
ii) Aufbringen des transmukosalen Systems auf die Schleimhaut einer Testperson, optional auf die Bukkalschleimhaut; und,
iii) Beibehalten des Kontakts des transmukosalen Systems mit der Schleimhautmembran für eine therapeutisch wirksame Zeitspanne; und
iv) optional die Entfernung des transmukosalen Systems, wenn ein gewünschter therapeutischer Effekt erzielt wurde.

17. Formulierung zur Verwendung nach Anspruch 16, wobei das transmukosale System ein Haftmaterial beinhaltet, wobei die Formulierung und das Haftmaterial gegebenenfalls in getrennten Kammern vorhanden sind.

18. Verfahren zur Herstellung eines bukkalen Arzneimittelabgabesystems, umfassend:
i) Herstellen einer Arzneimittelfreisetzungsschicht, umfassend die Formulierung, das transmukosale Arzneimittelabgabesystem oder die Arzneimitteldosierungsform zur oralen transmukosalen Verabreichung nach einem der Ansprüche 1 bis 15;
ii) Herstellen einer Klebeschicht; und,
iii) Zusammenfügen der Arzneimittelschicht und der Haftschichten auf einer Trägerschicht.

19. Arzneimitteldosierungsform zur Erhöhung der Permeation von Fenretinid oder eines pharmazeutisch akzeptablen Salzes davon in die Schleimhaut einer Testperson, die es benötigt, wobei die Arzneimitteldosierungsform hergestellt wird durch:
i) Beimischen von Fenretinid oder einem pharmazeutisch akzeptablen Salz davon mit einem Mittel zur Erhöhung der Permeation, umfassend eines oder mehrere von Propylenglykol und Menthol, und
ii) Formen der Beimischung zu einer Arzneimittelfreisetzungsschicht.

20. Verfahren zur Herstellung der Formulierung nach einem der Ansprüche 1 bis 15, umfassend:
i) Mischen einer Menge von zumindest einem Solubilisator, zumindest einem mucoadhäsiven Polymer und zumindest einem Permeationserhöher in einem Lösungsmittel zur Bildung eines Lösungsmittelgemisches:
ii) Zugabe einer Menge Fenretinid oder eines pharmazeutisch akzeptablen Salzes davon zu dem Lösungsmittelgemisch von Schritt i); und optional Anpassen eines Volumens davon auf 10 ml mit dem Lösungsmittelgemisch von Schritt i);
iii) Bilden einer Schicht aus der Mischung aus Fenretinid oder einem pharmazeutisch akzeptablen Salz davon aus Schritt ii); und,
iv) Trocknen der Schicht aus Schritt iii).

21. Mucoadhäsives Pflaster, umfassend die Formulierung nach einem der Ansprüche 1 bis 11.

## Revendications

1. Formulation, comprenant : au moins un matériau mucoadhésif : au moins une quantité efficace de fenrétinide ou un sel pharmaceutiquement acceptable de celui-ci ; et au moins un agent stimulateur de perméation transmuqueuse comprenant :
du propylène glycol (PG) et du menthol ; et au moins un agent solubilisant.

2. Formulation selon la revendication 1, dans laquelle le fenrétinide ou un sel pharmaceutiquement acceptable de celui-ci est présent à environ 0,1 % en poids jusqu'à environ 5 % en poids.

3. Formulation selon la revendication 1 ou 2, dans laquelle la formulation comprend 5 % en poids de fenrétinide ou un sel pharmaceutiquement acceptable de celui-ci.

4. Formulation selon la revendication 1, 2 ou 3, dans laquelle l'agent stimulateur de perméation transmuqueuse comprend d'environ 1 % en poids à environ 2,5 % en poids de PG, et d'environ 1 % en poids à environ 5 % en poids de menthol.

5. Formulation selon l'une quelconque des revendications 1 à 4, dans laquelle l'au moins un agent solubilisant comprend un ou plusieurs parmi l'acide désoxycholique, un ester d'acide gras supérieur de sorbitane de polyoxyéthylène et des combinaisons de ceux-ci.

6. Formulation selon la revendication 5, dans laquelle l'ester d'acide gras supérieur de sorbitane de polyoxyéthylène est choisi parmi le polysorbate 20, le polysorbate 80 et des combinaisons de ceux-ci.

7. Formulation selon l'une quelconque des revendications 1 à 4, dans laquelle l'au moins un agent solubilisant comprend un ou plusieurs parmi le polysorbate 80 et le désoxycholate de sodium.

8. Formulation selon l'une quelconque des revendications précédentes, dans laquelle le fenrétinide ou un sel pharmaceutiquement acceptable de celui-ci et l'au moins un agent stimulateur de perméation sont adaptés pour être en contact avec au moins une membrane muqueuse commune, facultativement dans laquelle la membrane muqueuse est la muqueuse buccale.

9. Formulation selon l'une quelconque des revendications précédentes, se présente comme un produit de bouche tel qu'un dentifrice, un bain de bouche ou un rince-bouche, un gel ou une pâte, un spray, un chewing-gum et/ou une pastille.

10. Formulation selon l'une quelconque des revendications précédentes, dans laquelle la formulation contient une quantité prédéterminée de fenrétinide ou un sel pharmaceutiquement acceptable de celui-ci dans une quantité choisie dans le groupe constitué de 10 µg, 15 µg, 25 µg, 50 µg, 100 µg, 500 µg, 1 mg, 2 mg, 3 mg, 4 mg, 5 mg, 6 mg, 7 mg, 8 mg, 9 mg et 10 mg de matériau adhésif, le matériau adhésif fournissant une adhérence à la membrane muqueuse orale d'un sujet.

11. Formulation selon l'une quelconque des revendications précédentes, dans laquelle la quantité du fenrétinide ou d'un sel pharmaceutiquement acceptable de celui-ci absorbé par la muqueuse orale est choisie dans le groupe constitué d'au moins 35 %, d'au moins 40 %, d'au moins 45 %, d'au moins 50 %, d'au moins 55 %, d'au moins 60 %, d'au moins 65 %, d'au moins 70 %, d'au moins 75 %, et d'au moins 80 %, d'au moins 85 %, d'au moins 90 %, d'au moins 95 %, d'au moins 98 % et d'au moins 99 % du médicament dans la forme posologique.

12. Système de distribution de médicament transmuqueux comprenant au moins une couche de libération de médicament comprenant la formulation selon l'une quelconque des revendications précédentes, au moins une couche adhésive, et au moins une couche de soutien.

13. Forme posologique de médicament pour administration transmuqueuse orale, comprenant :
une formulation comprenant : au moins une quantité efficace de fenrétinide ou d'un sel pharmaceutiquement acceptable de celui-ci, et au moins un agent stimulateur de perméation transmuqueuse choisi parmi le propylène glycol (PG) et le menthol ; et
un matériau adhésif, le matériau adhésif fournissant l'adhérence de la forme posologique du médicament à la membrane muqueuse orale.

14. Formulation, système de distribution de médicament transmuqueux ou forme posologique de médicament pour administration transmuqueuse orale selon l'une quelconque des revendications précédentes pour une utilisation dans un procédé de traitement et de prophylaxie d'un état cancéreux ou précancéreux, ladite utilisation comprenant : l'administration de ladite formulation à un sujet ayant besoin d'un tel traitement.

15. Formulation, système de distribution de médicament transmuqueux ou forme posologique de médicament pour administration transmuqueuse orale pour une utilisation selon la revendication 11, dans laquelle l'état cancéreux ou précancéreux comprend un ou plusieurs parmi : carcinome à cellules squameuses orales, lésions dysplasiques intra-orales, carcinome à cellules squameuses de la tête et du cou.

16. Formulation selon l'une quelconque des revendications 1 à 11 pour une utilisation dans un procédé, comprenant :
i) la fourniture d'un système transmuqueux comprenant ladite formulation ;
ii) l'application du système transmuqueux à une membrane muqueuse d'un sujet, facultativement la muqueuse buccale ; et,
iii) le maintien du système transmuqueux en contact avec la membrane muqueuse pendant une période de temps thérapeutiquement efficace ; et,
iv) facultativement le retrait du système transmuqueux lorsqu'un effet thérapeutique souhaité a été obtenu.

17. Formulation pour une utilisation selon la revendication 16, dans laquelle le système transmuqueux comporte un matériau adhésif, facultativement dans laquelle la formulation et le matériau adhésif sont présents dans des compartiments séparés.

18. Procédé de fabrication d'un système de distribution de médicament buccal, comprenant :
i) la préparation d'une couche de libération de médicament comprenant la formulation, le système de distribution de médicament transmuqueux ou la forme posologique du médicament pour l'administration transmuqueuse orale selon l'une quelconque des revendications 1 à 15 ;
ii) la préparation d'une couche adhésive ; et,
iii) l'assemblage de la couche de médicament et des couches adhésives sur une couche de soutien.

19. Forme posologique de médicament pour l'augmentation de la perméation du fenrétinide ou d'un sel pharmaceutiquement acceptable de celui-ci dans une muqueuse d'un sujet qui en a besoin, la forme posologique de médicament étant préparée par :
i) le mélange de fenrétinide ou d'un sel pharmaceutiquement acceptable de celui-ci avec un agent stimulateur de perméation comprenant un ou plusieurs parmi le propylène glycol et le menthol, et
ii) la formation du mélange dans une couche de libération de médicament.

20. Procédé de fabrication de la formulation selon l'une quelconque des revendications 1 à 15, comprenant :
i) le brassage d'une quantité d'au moins un solubilisant, d'au moins un polymère muco-adhésif, et d'au moins un stimulateur de perméation dans un solvant pour former un mélange de solvant :
ii) l'ajout d'une quantité de fenrétinide ou d'un sel pharmaceutiquement acceptable de celui-ci au mélange de solvant de l'étape i) ; et facultativement l'ajustement d'un volume de celui-ci à 10 ml avec le mélange de solvant de l'étape i) ;
iii) la formation d'une couche du fenrétinide ou d'un mélange pharmaceutiquement acceptable de sel de celui-ci de l'étape ii) ; et,
iv) le séchage de la couche de l'étape iii).

21. Patch muco-adhésif comprenant la formulation selon l'une quelconque des revendications 1 à 11.
